# EUROPÄISCHE PATENTSCHRIFT

(11) **EP 3 307 681 B1**
(45) Veröffentlichungstag und Bekanntmachung des Hinweises auf die Patenterteilung: **17.04.2019**
(21) Anmeldenummer: 16728011.4
(22) Anmeldetag: 09.06.2016
(51) Int. Cl.: C01C 1/04, C01B 3/02

(54) **MEHRDRUCKVERFAHREN ZUR HERSTELLUNG VON AMMONIAK**
MULTIPLE-PRESSURE PROCESS FOR THE PRODUCTION OF AMMONIA
PROCÉDÉ À PRESSION MIXTE DE PRODUCTION D'AMMONIAC

(30) Priorität: 12.06.2015 DE 102015210801
(43) Veröffentlichungstag der Anmeldung: 18.04.2018
(73) Patentinhaber: thyssenkrupp Industrial Solutions AG, 45143 Essen (DE); thyssenkrupp AG, 45143 Essen (DE)
(72) Erfinder: GORVAL, Evgeni, 44135 Dortmund (DE); HEUN, Reinhard, 58313 Herdecke (DE); JOHANNING, Joachim, 46045 Oberhausen (DE); NÖLKER, Klaus, 44265 Dortmund (DE)
(74) Vertreter: thyssenkrupp Intellectual Property GmbH
(86) Internationale Anmeldenummer: PCT/EP2016/063088
(87) Internationale Veröffentlichungsnummer: WO 2016/198487

(56) Entgegenhaltungen:
- EP-B1- 1 339 641
- DD-A3- 225 029
- DE-A1-102011 016 759
- DE-C1- 4 304 567
- US-A1- 2010 150 810
- US-A1- 2012 070 364

## Beschreibung

Die Erfindung betrifft ein Verfahren und eine Vorrichtung zur Herstellung von Ammoniak auf unterschiedlichen Druckniveaus.

Bei der Herstellung von Ammoniak enthält das Synthesegas neben Wasserstoff und Stickstoff zusätzlich üblicherweise Inertgase wie Methan und Edelgase, welche die Ausbeute an Ammoniak beeinträchtigen. Bei diesen Verfahren wird üblicherweise zunächst frisches Synthesegas mehrstufig auf hohen Druck verdichtet. Dann wird das verdichtete, frische Synthesegas in einen Kreislauf eingespeist, der durch einen oder mehrere katalysatorgefüllte Reaktoren geführt wird, in denen Ammoniak erzeugt wird. Im Kreislauf ist ein Abscheidesystem vorgesehen, mit dem das erzeugte Ammoniak aus dem Kreislauf in flüssiger Form entnommen wird.

Die Inertgase sind in Ammoniak nur in geringer Konzentration löslich und werden daher nur zu einem geringen Teil zusammen mit dem Ammoniak entnommen. Um eine Anreicherung der Inertgase im Kreislauf zu vermeiden, wird bei diesen herkömmlichen Verfahren beständig ein Teil des im Kreislauf geführten Gases als Spülgas (*Purge*) ausgeschleust. Aus diesem ausgeschleusten Spülgas werden anschließend Reste von Ammoniak ausgewaschen. Wasserstoff sowie ggf. auch Stickstoff werden abgeschieden und zurückgewonnen, beispielsweise über Membranen oder durch Zerlegung bei tiefen Temperaturen. Die verbleibenden Inertgase, insbesondere Methan, Argon und Helium werden verworfen oder anderweitig verwertet, insbesondere zu Heizzwecken. Der zurückgewonnene Wasserstoff sowie ggf. auch der zurückgewonnene Stickstoff werden dem frischen Synthesegas vor der Verdichtung beigemischt und auf diese Weise verwertet.

DE 100 57 863 A1 und DD 225 029 A3 offenbaren Verfahren zur Herstellung von Ammoniak aus frischem Synthesegas, welches außer Wasserstoff und Stickstoff inerte Bestandteile enthält, in mindestens zwei Reaktionssystemen, wobei die Synthese von Ammoniak aus Synthesegas nacheinander in verschiedenen Synthesesystemen erfolgt. Dabei werden Inertgasbestandteile über einen Spülgasstrom (*Purge*) abgetrennt und ausgeschleust.

Es ist energetisch jedoch ungünstig, Spülgas aus dem Kreislauf auszuschleusen, da somit große Mengen an Gas bei der Trennung einem Druckverlust unterworfen werden und danach wieder aufwändig rückverdichtet werden müssen. Aus diesem Grund wird häufig eine Anreicherung der Inertgase von ursprünglich 1 bis 2 Vol.-% im frischen Synthesegas bis auf 10 bis 20 Vol.-% innerhalb des im Kreislauf geführten Gases in Kauf genommen, obwohl damit zwangsläufig der Nachteil verbunden ist, dass die Partialdrücke von Wasserstoff und Stickstoff beträchtlich niedriger liegen als in einem Synthesekreislauf, welcher weniger oder keine Inertgase enthält. Aus diesem Grund müssen auch die Katalysatorvolumina und die sie enthaltenden Reaktoren deutlich größer dimensioniert werden, als dies ohne Inertgase im Synthesekreislauf erforderlich wäre.

Bei der Ammoniaksynthese entsteht im Reaktor aus dem Synthesegas ein Gasgemisch, welches neben dem nicht umgesetzten Wasserstoff und Stickstoff das gebildete Ammoniak sowie die Inertgase enthält. Am Ausgang des Reaktors liegt das erzeugte Ammoniak gasförmig vor. Um das Ammoniak vom Produktgas zu trennen, wird es kondensiert, damit es flüssig aus dem Kreislauf abgezogen werden kann. Da der Taupunkt des Ammoniaks von dessen Partialdruck abhängt, wird die Kondensation von Ammoniak begünstigt durch einen hohen Synthesedruck, eine hohe Ammoniakkonzentration und eine niedrige Temperatur. Eine hohe Ammoniakkonzentration lässt sich mit großen Katalysatorvolumina sowie geringen Konzentrationen an Inertgas erzielen. Ein hoher Synthesedruck bedeutet entsprechenden Energieaufwand für die Synthesegasverdichtung und eine niedrige Abkühltemperatur setzt die entsprechenden Kühlvorrichtungen für das im Kreislauf geführte Gas voraus.

Um das erzeugte Ammoniak innerhalb des Kreislaufes kondensieren zu können, wird üblicherweise ein Synthesedruck im Bereich von 150 und 280 bar gewählt. Dieser relativ hohe Druck bietet neben den verbesserten Reaktionsbedingungen den Vorteil, dass ein Großteil des Ammoniaks schon bei relativ hohen Temperaturen kondensiert, wie sie teilweise sogar mittels Wasserkühlung erreicht werden können. Da das im Kreislauf geführte Gas, welches zum Reaktor zurückgeführt wird, eine möglichst geringe Ammoniackonzentration aufweisen sollte, wird der Wasserkühlung üblicherweise ein zusätzlicher Tiefkühlkreislauf nachgeschaltet, um mit noch niedrigeren Temperaturen weiteres Ammoniak auszukondensieren.

Je niedriger der Synthesedruck liegt, umso mehr verkleinert sich der Anteil der Wärme, der mittels Wasser- oder Luftkühlung abgeführt werden kann und derjenige Anteil an Wärme, der mittels Tiefkühlung abzuführen ist, vergrößert sich entsprechend. Während der Verdichtungsaufwand für den Synthesekreislauf mit abnehmendem Synthesedruck abnimmt, steigt der Verdichtungsaufwand für den Kältekreislauf, weil mehr Kälte für die Abscheidung des im Synthesekreislauf erzeugten Ammoniaks benötigt wird. Bei der Ammoniaksynthese bei geringerem Synthesedruck wird der Kondensationsanteil vor der Tiefkühlung dadurch erhöht, dass ein sehr niedriger Gehalt an Inertgas über einen hohen Spülgasstrom *(Purge)* eingestellt wird. Auch hier erhöht ein geringerer Gehalt an Inertgas die Ammoniackonzentration und somit den Taupunkt.

DE 10 055 818 A1 offenbart ein Verfahren zur katalytischen Herstellung von Ammoniak aus einem Stickstoff-Wasserstoff-Gasgemisch umfassend die Bildung von Synthesegas aus Erdgas und sauerstoffreichem Gas in einem autothermen Reaktor, die katalytische Umwandlung von Kohlenmonoxid in Wasserstoff, die Entfernung von Kohlenmonoxid, Kohlendioxid und Methan sowie das Zuführen des erhaltenen Stickstoff-Wasserstoff-Gasgemischs zu einer katalytischen Synthese von Ammoniak.

US2010/0150810 offenbart ein Verfahren zur Herstellung von Ammoniak, wobei Synthesegas in einem autothermen Reformer generiert wird.

Der Erfindung liegt die Aufgabe zugrunde, ein verbessertes Verfahren zur Herstellung von Ammoniak zur Verfügung stellen.

Ein erster Aspekt der Erfindung betrifft ein Verfahren zur Herstellung von Ammoniak umfassend die Schritte von Anspruch 1. In einer Ausführungsform umfasst das Verfahren die Schritte:
(a) Bereitstellen eines Gasgemischs umfassend Wasserstoff, Stickstoff, Wasser, Methan, Kohlenmonoxid, Kohlendioxid, sowie ggf. Argon und/oder ggf. Helium;
(b) Abtrennen zumindest eines Teils des Wassers, ggf. Abtrennen zumindest eines Teils des Methans, ggf. zumindest eines Teils des Argons, Abtrennen eines Teils des Kohlenmonoxids und zumindest eines Teils des Kohlendioxids von dem Gasgemisch, bevorzugt durch Abkühlen, CO-Konvertierung, ,CO2-Wäsche, Methanisierung und/oder Auswaschen und/oder Adsorption; ggf. Einstellen des für die Ammoniaksynthese benötigten Verhältnisses von Wasserstoff zu Stickstoff auf etwa 3;
(c) ggf. Verdichten des Gasgemischs auf erhöhten Druck, vorzugsweise auf einen Druck im Bereich von 60 bis 130 bar, bevorzugter von 90 bis 115 bar; Synthetisieren von Ammoniak aus zumindest einem Teil des Wasserstoffs und aus zumindest einem Teil des Stickstoffs, welcher in dem Gasgemisch enthalten ist; und Abtrennen zumindest eines Teils des synthetisierten Ammoniaks von dem Gasgemisch durch Abkühlen;
(d) Verdichten des Gasgemischs auf einen Druck, welcher höher ist als der Druck in Schritt (c), vorzugsweise auf einen Druck im Bereich von 150 bis 280 bar;
   (d') ggf. entweder Abtrennen zumindest eines Teils des synthetisierten Ammoniaks von dem Gasgemisch, bevorzugt durch Abkühlen; oder ggf. Synthetisieren von Ammoniak aus zumindest einem Teil des Wasserstoffs und aus zumindest einem Teil des Stickstoffs, welcher in dem Gasgemisch enthalten ist, sowie Abtrennen zumindest eines Teils des synthetisierten Ammoniaks von dem Gasgemisch, bevorzugt durch Abkühlen;
(e) Vereinen des Gasgemischs mit einem im Kreislauf geführten Gasgemisch umfassend Wasserstoff, Stickstoff und Ammoniak sowie gegebenenfalls die in Schritt (b) nicht vollständig abgetrennten anderen Komponenten; dabei kann das Vereinen gemäß Schritt (e) bevorzugt nach oder bevorzugter vor der Verdichterstufe im Kreislauf erfolgen, welche das Gasgemisch bei dem erfindungsgemäßen Verfahren durchströmt;
(f) Synthetisieren von Ammoniak aus zumindest einem Teil des Wasserstoffs und aus zumindest einem Teil des Stickstoffs, welcher in dem Gasgemisch enthalten ist; Abtrennen zumindest eines Teils des synthetisierten Ammoniaks von dem Gasgemisch durch Abkühlen; Verdichten des Gasgemischs auf einen erhöhten Druck; und Zurückführen des Gasgemischs als im Kreislauf geführtes Gasgemisch zu Schritt (e),
wobei das in Schritt (a) bereitgestellte Gasgemisch durch Reformierung von Kohlenwasserstoffen hergestellt wird, wobei die Reformierung in einem autothermen Reformer (ATR) erfolgt, welcher mit reinem Sauerstoff oder mit an Sauerstoff angereicherter Luft oder mit Luft betrieben wird.

In dem erfindungsgemäßen Verfahren wird die Technologie der Gewinnung eines Ausgangsgasgemisches für die Ammoniakherstellung mittels eines ATR mit der Ammoniaksynthese auf mehreren Druckstufen kombiniert. Dadurch lassen sich Anlagenkapazitäten schaffen, die über denjenigen herkömmlicher Anlagen liegen, beispielsweise Anlagenkapazitäten von mehr als 3.300 Tagestonnen.

Ein Merkmal der konventionellen Ammoniak-Harnstoff-Anlagen ist eine Überproduktion von Ammoniak verglichen mit CO₂, so dass nie genug CO₂ produziert wird, um das produzierte Ammoniak vollständig zu Harnstoff umzusetzen. Ein wesentlicher Vorteil der NH₃-Anlagen, die gemäß der vorliegenden Erfindung ein Ausgangsgasgemisch aus einem autothermen Reformer verwenden, liegt demgegenüber in einer gesteigerten und steuerbaren Produktion von CO₂. In Verbindung mit der Ammoniaksynthese auf mehreren Druckniveaus, die eine sehr hohe NH₃-Produktion zulässt, macht dies auch eine Maximierung der Harnstoffproduktion möglich.

In Schritt (a) des erfindungsgemäßen Verfahrens wird ein Gasgemisch bereitgestellt, welches Wasserstoff, Stickstoff, Wasser (Dampf), Methan, Kohlenmonoxid, Kohlendioxid und ggf. Argon umfasst, und ggf. Spuren von Helium. Das Gasgemisch wird bevorzugt als Synthesegas aus Kohlenwasserstoffen, vorzugsweise aus Erdgas, Wasser in Form von Dampf und Luft bzw. Sauerstoff durch Reformierung gewonnen. Geeignete Verfahren zur Erzeugung eines solchen Synthesegases sind einem Fachmann bekannt und es kann diesbezüglich beispielsweise vollumfänglich verwiesen werden auf A. Nielsen, I. Dybkjaer, Ammonia - Catalysis and Manufacture, Springer Berlin 1995, Kapitel 6, Seiten 202-326. Der volumenanteilige Hauptbestandteil dieses Gasgemischs ist bevorzugt Wasserstoff, wobei der Stickstoffanteil ggf. auch relativ hoch sein kann, abhängig davon, ob bei der Herstellung Luft, mit Sauerstoff angereicherte Luft oder sogar reiner Sauerstoff eingesetzt wurde.

Das in Schritt (a) des erfindungsgemäßen Verfahrens bereitgestellte Gasgemisch kann als Synthesegas bereits herkömmlichen Aufbereitungsmaßnahmen unterzogen worden sein, wie z.B. Heliumentfernung und Erdgasentschwefelung.

In einer bevorzugten Ausführungsform wird das in Schritt (a) bereitgestellte Gasgemisch aus Kohlenwasserstoffen, vorzugsweise aus Erdgas, Wasser in Form von Dampf und Luft bzw. Sauerstoff durch Reformierung gewonnen, wobei die Kohlenwasserstoffe bevorzugt kein Argon oder nur eine geringe Menge an Argon aufweisen. Es ist einem Fachmann bekannt, dass Erdgas in verschiedenen Regionen häufig auch verschiedene Mengen an Argon und ggf. gar kein Argon enthält. Erfolgt die Reformierung mit Hilfe von reinem Sauerstoff, z.B. aus einer Luftzerlegungsanlage, oder mit an Sauerstoff in hohem Maße angereicherter Luft, so weisen diese bevorzugt ebenfalls kein Argon oder nur eine geringe Menge an Argon auf, so dass auch auf diesem Wege keine signifikanten Mengen an Argon eingetragen werden. Bei dieser Ausführungsform enthält das in Schritt (a) bereitgestellte Gasgemisch daher allenfalls nur geringe Mengen an Argon und in Schritt (b) ist die Abtrennung von Argon aus dem Gasgemisch ggf. nicht erforderlich - in Schritt (b) erfolgt dann im Wesentlichen die Abtrennung der übrigen Gase, insbesondere zumindest eines Teils des Kohlenmonoxids sowie des Kohlendioxids und ggf. zumindest eines Teils des Methans.

In einer anderen bevorzugten Ausführungsform wird das in Schritt (a) bereitgestellte Gasgemisch aus Kohlenwasserstoffen, vorzugsweise aus Erdgas, Wasser in Form von Dampf und Luft bzw. Sauerstoff durch Reformierung gewonnen, wobei die Kohlenwasserstoffe bevorzugt bereits eine signifikante Menge an Argon aufweisen. Erfolgt die Reformierung zudem mit Hilfe von Luft oder mit an Sauerstoff in nur geringem Maße angereicherter Luft, so wird darüber zusätzliches Argon eingetragen. Bei dieser Ausführungsform enthält das in Schritt (a) bereitgestellte Gasgemisch daher signifikante Mengen an Argon und in Schritt (b) erfolgt dann die Abtrennung zumindest eines Teils des Argons sowie des Kohlenmonoxids und des Kohlendioxids sowie ggf. die Abtrennung zumindest eines Teils des Methans.

Das in Schritt (a) des erfindungsgemäßen Verfahrens bereitgestellte Gasgemisch wird als Synthesegas in Schritt b) herkömmlichen Aufbereitungsmaßnahmen unterzogen, wie z.B. Konvertierung von Kohlenmonoxid zu Kohlendioxid und Kohlendioxidwäsche. Auch nach Durchführung dieser Aufbereitungsmaßnahmen umfasst das Gasgemisch jedoch Wasserstoff, Stickstoff, Wasser (Dampf), Methan und ggf. Argon, üblicherweise auch noch andere Bestandteile wie Restmengen an Kohlenmonoxid, Restmengen an Kohlendioxid und ggf. Spuren von Helium. Erfolgt die weitere Abtrennung in Schritt (b) mit Hilfe kryogener Methoden, wie z. B. durch Stickstoffwäsche oder starke Abkühlung (vgl. US 3,442,613) so werden vorher in Schritt (b) Reste von Kohlenmonoxid, und dabei auch ggf. Kohlendioxid, bevorzugt durch Methanisierung weitgehend zu Methan umgesetzt. Zur Abtrennung von Kohlenmonoxid durch starke Abkühlung und Kondensation sind besonders tiefe Temperaturen erforderlich, so dass ggf. vorhandenes Kohlendioxid beim Abkühlen bereits vorher kondensiert. Erfolgt die Abtrennung in Schritt (b) dagegen durch Druckwechsel-Adsorption (PSA), so kann bevorzugt auf die Methanisierung verzichtet werden. Geeignete Verfahren zur Hydrierung von Kohlenmonoxid zu Methan sind einem Fachmann bekannt. Durch die Methanisierung wird der Gehalt an Kohlenmonoxid, und dabei auch ggf. Kohlendioxid, in dem Gasgemisch verringert und der Gehalt an Methan in dem Gasgemisch erhöht. Dies hat einerseits den Vorteil, dass Kohlenmonoxid wie auch Kohlendioxid als Katalysatorgift ohnehin nur in sehr geringen Mengen bei der Ammoniaksynthese in dem Gasgemisch enthalten sein darf. Andererseits hat aber auch Methan bei Atmosphärendruck einen um ca. 30°C höheren Siedepunkt, so dass Methan im bevorzugten Schritt (b) durch geringeren Aufwand von dem Gasgemisch abgetrennt werden kann als Kohlenmonoxid.

Im bevorzugten Schritt (b) des erfindungsgemäßen Verfahrens erfolgt bevorzugt das Abtrennen zumindest eines Teils des Wassers, zumindest eines Teils des Methans, ggf. zumindest eines Teils des Argons und ggf. zumindest eines Teils des Kohlenmonoxids und ggf. zumindest eines Teils des Kohlendioxids von dem Gasgemisch, bevorzugt durch Abkühlen und/oder Auswaschen. Dabei wird das Gasgemisch bevorzugt so stark abgekühlt, dass Wasser, Methan und ggf. Argon unter den gegebenen Bedingungen auskondensieren und so von der Gasphase durch Phasentrennung abgetrennt werden können.

Wird in Schritt (b) von dem Gasgemisch zumindest ein Teil des vorhandenen Kohlenmonoxids abgetrennt, so wird dieses Kohlenmonoxid in einer bevorzugten Ausführungsform einem CO-Konverter zugeführt, in dem das Kohlenmonoxid zu Kohlendioxid oxidiert und Wasser zu Wasserstoff reduziert wird. Geeignete CO-Konverter sind einem Fachmann bekannt. In einer anderen bevorzugten Ausführungsform wird das abgetrennte Kohlenmonoxid nicht unmittelbar einem CO-Konverter zugeführt, sondern dem Edukt beigemischt, aus dem das in Schritt (a) bereitgestellte Gasgemisch hergestellt wird. Handelt es sich bei dem Edukt um Erdgas, so dass das in Schritt (a) bereitgestellte Gasgemisch aus Erdgas gewonnenes Synthesegas ist, so wird das abgetrennte Kohlenmonoxid bevorzugt dem Erdgas zugemischt und die so erhaltene Mischung anschließend verdichtet, ehe daraus durch Reformierung das Synthesegas gewonnen wird.

In einer bevorzugten Ausführungsform erfolgt die Abtrennung durch Abkühlung mit Hilfe kryogener Methoden, besonders bevorzugt durch Stickstoffwäsche. Diese Ausführungsform ist insbesondere dann bevorzugt, wenn das in Schritt (a) bereitgestellte Gasgemisch Argon enthält. Das Gasgemisch wird bevorzugt auf eine Temperatur abgekühlt, bei der Wasser, Methan, Argon und ggf. Kohlenmonoxid unter den gegebenen Bedingungen nicht mehr gasförmig sind, Wasserstoff hingegen schon. Das Gasgemisch wird bevorzugt auf Temperaturen von weniger als -150°C, bevorzugter weniger als -170°C und insbesondere ca. -190°C abgekühlt. Unter diesen Bedingungen kondensieren Kohlenmonoxid und Methan zumindest teilweise aus; Wasser und Kohlendioxid werden bereits bei deutlich höheren Temperaturen fest. In einer bevorzugten Ausführungsform erfolgt die Abtrennung durch Stickstoffwäsche bei einem Druck des Gasgemischs im Bereich von 30 bis 100 bar, bevorzugt -90 bar. Dies hat den Vorteil, dass dann nicht so tiefe Temperaturen erreicht werden müssen, um das Auskondensieren der abzutrennenden Gase zu bewirken. Darüber hinaus hat dies den Vorteil, dass das Gasgemisch bei diesem Druck ggf. auch ohne zusätzliche Verdichtung direkt der Ammoniaksynthese in Schritt (c) zugeführt werden kann, d.h. auch einen weiteren Verdichter, welcher dem Ammoniakreaktor vorgeschaltet ist, ggf. verzichtet werden kann. Die Stickstoffwäsche hat den Vorteil, dass die Abtrennung der Gase praktisch vollständig oder fast vollständig erfolgen kann.

Erfolgt die Abtrennung durch eine Stickstoffwäsche, wird das Gasgemisch bevorzugt zunächst konventionell abgekühlt, beispielsweise in einem Wärmetauscher. Ggf. wird das Gasgemisch zunächst stark (z.B. auf -185 °C) abgekühlt (vgl. US 3,442,613), um dann anschließend einer Stickstoffwäsche unterzogen zu werden.

In einer anderen bevorzugten Ausführungsform erfolgt die Abtrennung nur durch starke Abkühlung (vgl. US 3,442,613), d.h. es erfolgt kein Auswaschen wie bei der Stickstoffwäsche, sondern eine starke Unterkühlung, wobei Methan praktisch vollständig und ggf. Argon zumindest teilweise durch die Kondensation auskondensiert werden. Diese Ausführungsform ist insbesondere dann bevorzugt, wenn das in Schritt (a) bereitgestellte Gasgemisch kein oder nur eine geringe Menge an Argon enthält. Im Fall eines mit Sauerstoff betriebenen autothermen Reformers bzw. Sekundärreformers enthält das Gasgemisch (Synthesegas) kein oder nur eine geringe Menge an Argon, sofern im Erdgas, welches zur Erzeugung des Gasgemischs (Synthesegases) eingesetzt wird, kein oder nur eine geringe Menge an Argon enthalten ist, so dass durch starke Abkühlung bereits praktisch alle Inertgase, d.h. Methan, ggf. Kohlenmonoxid und ggf. Kohlendioxid entfernt werden können.

Erfolgt die Abtrennung durch starke Abkühlung (vgl. US 3,442,613), wird das Gasgemisch bevorzugt zunächst konventionell abgekühlt, beispielsweise in einem Wärmetauscher.

In einer weiteren bevorzugten Ausführungsform erfolgt die Abtrennung durch Druckwechsel-Adsorption (*Pressure Swing Adsorption,* PSA). Die Druckwechsel-Adsorption ist einem Fachmann bekannt. Dabei werden vorzugsweise Molekularsiebe eingesetzt, welche im Wesentlichen nur Wasserstoff und ggf. Spuren an Argon durchlassen, häufig ∼ 50 ppm. Diese Ausführungsform ist ebenfalls insbesondere dann bevorzugt, wenn das in Schritt (a) bereitgestellte Gasgemisch kein oder nur eine geringe Menge an Argon enthält.

Erfolgt die Abtrennung durch eine Druckwechsel-Adsorption, wird bevorzugt auf eine Methanisierung von im Gasgemisch befindlichem Kohlenmonoxid und/oder Kohlendioxid verzichtet.

Das durch den bevorzugten Schritt (b) erhaltene Gasgemisch weist beispielsweise einen Gehalt an Wasser von höchstens 0,05 Mol.-%, bevorzugter höchsten 0,02 Mol.-% und insbesondere höchstens 0,01 Mol.-%; und/oder
Gehalt an Kohlendioxid von höchstens 5 ppm vol., bevorzugter höchsten 2 ppm vol. und insbesondere höchstens 1 ppm vol.; und/oder
Gehalt an Kohlenmonoxid von höchstens 5 ppm vol., bevorzugter höchsten 2 ppm vol. und insbesondere höchstens 1 ppm vol.; und/oder
Gehalt an Methan von bis zu 0,05 Mol.-%, bevorzugter höchsten 0,02 Mol.-% und insbesondere höchstens 0,01 Mol.-%; und/oder
Gehalt an Argon von höchstens 0,05 Mol.-%, bevorzugter höchsten 0,02 Mol.-% und insbesondere höchstens 0,01 Mol.-% auf.

Die zuvor angegebenen Obergrenzen für Methan und Argon gelten nur für die inertenfreie Synthese, bei der kein Ausschleusen von Spülgas erfolgt, welches Inertgase enthält (ohne Purge). Bei der im Rahmen der Erfindung alternativ möglichen Variante mit Purge gelten diese Obergrenzen nicht.

Je nach Verhältnis von Wasserstoff und Stickstoff in dem durch Schritt (b) erhaltenen Gasgemisch umfasst Schritt (b) ggf. zusätzlich das Einstellen des für die Ammoniaksynthese benötigten Verhältnisses von Wasserstoff zu Stickstoff auf etwa 3.

In Schritt (c) des erfindungsgemäßen Verfahrens wird das Gasgemisch bevorzugt auf erhöhten Druck verdichtet, vorzugsweise auf einen Druck im Bereich von 60 bis 130 bar, bevorzugter 90 bis 115 bar. In einer bevorzugten Ausführungsform erfolgt sogar bereits eine Verdichtung auf einen Druck im Bereich von 150 bis 180 bar. Dieser Druck ist dann der Synthesedruck, mit dem das Gasgemisch in einen Ammoniakreaktor geleitet wird. Es ist erfindungsgemäß jedoch ebenso möglich, in Anbetracht des ggf. ohnehin bereits vergleichsweise hohen Drucks des in Schritt (b) bereitgestellten Gasgemischs (*FrontEnd*), das Gasgemisch ohne zusätzliche Verdichtung dem Ammoniakreaktor zuzuführen. In diesem Ammoniakreaktor wird Ammoniak synthetisiert aus zumindest einem Teil des Wasserstoffs und aus zumindest einem Teil des Stickstoffs, welcher in dem Gasgemisch enthalten ist. Vorzugsweise weist der Ammoniakreaktor zumindest ein Katalysatorbett auf, welches von dem Gasgemisch nicht rein axial, sondern vorwiegend radial durchströmt wird, vorzugsweise von außen nach innen. Bevorzugt wird dabei das Katalysatorbett nicht gekühlt, sondern die Synthese adiabat geführt. Danach wird zumindest ein Teil des Ammoniaks von dem Gasgemisch durch Abkühlen abgetrennt, wozu das Gasgemisch bevorzugt zunächst einen Wärmetauscher und anschließend eine Kondensationsvorrichtung durchläuft. Dabei wird das Gasgemisch abgekühlt, bevorzugt auf Temperaturen von weniger als -15°C, bevorzugter weniger als -25°C und insbesondere ca. -35°C, bevorzugt allerdings nicht weniger als -79°C, so dass Ammoniak unter den gegebenen Bedingungen auskondensiert und so von der Gasphase durch Phasentrennung abgetrennt werden kann.

In Schritt (d) des erfindungsgemäßen Verfahrens wird das Gasgemisch auf einen Druck verdichtet, welcher höher ist als der Druck in Schritt (c), vorzugsweise auf einen Druck im Bereich von 150 bis 280 bar. In einer anderen bevorzugten Ausführungsform ist der Druck in Schritt (d) höher als 100 bar. In einer besonders bevorzugten Ausführungsform sind sowohl der Druck in Schritt (c) als auch der Druck in Schritt (d) jeweils höher als 100 bar, wobei der Druck in Schritt (d) höher als der Druck in Schritt (c) ist.

In einer bevorzugten Ausführungsform kann Schritt (d) des erfindungsgemäßen Verfahrens als Teilschritt (d') nach dem Verdichten des Gasgemischs zwei bevorzugte Alternativen umfassen:
In einer ersten bevorzugten Alternative umfasst Teilschritt (d') das Abtrennen zumindest eines Teils des synthetisierten Ammoniaks von dem Gasgemisch, bevorzugt durch Abkühlen.

In einer zweiten bevorzugten Alternative umfasst Teilschritt (d') das Synthetisieren von Ammoniak aus zumindest einem Teil des Wasserstoffs und aus zumindest einem Teil des Stickstoffs, welcher in dem Gasgemisch enthalten ist, sowie das Abtrennen zumindest eines Teils des synthetisierten Ammoniaks von dem Gasgemisch, bevorzugt durch Abkühlen. Dazu umfasst Teilschritt (d') dann das Einleiten des Gasgemischs in einen weiteren Ammoniakreaktor, in dem Ammoniak aus zumindest einem Teil des Wasserstoffs und aus zumindest einem Teil des Stickstoffs, welcher in dem Gasgemisch enthalten ist, synthetisiert wird. Vorzugsweise weist der weitere Ammoniakreaktor zumindest ein Katalysatorbett auf, welches von dem Gasgemisch nicht axial, sondern vorwiegend radial durchströmt wird, vorzugsweise von außen nach innen. Bevorzugt wird dabei das Katalysatorbett nicht gekühlt, sondern die Synthese adiabat geführt.

Bei beiden bevorzugten Alternativen wird bevorzugt zumindest ein Teil des Ammoniaks von dem Gasgemisch durch Abkühlen abgetrennt, wozu das Gasgemisch bevorzugt zunächst mindestens einen Wärmetauscher und anschließend eine Kondensationsvorrichtung durchläuft. Dabei wird das Gasgemisch abgekühlt. Das Gasgemisch wird bevorzugt auf Temperaturen von weniger als -15°C, bevorzugter weniger als -25°C und insbesondere ca. - 35°C abgekühlt, bevorzugt allerdings nicht weniger als -79°C, so dass Ammoniak unter den gegebenen Bedingungen auskondensiert und so von der Gasphase durch Phasentrennung abgetrennt werden kann.

In Schritt (e) des erfindungsgemäßen Verfahrens wird das Gasgemisch mit einem im Kreislauf geführten Gasgemisch umfassend Wasserstoff, Stickstoff und Ammoniak sowie gegebenenfalls in Schritt (b) nicht vollständig abgetrennte, andere Komponenten, vereint.

In einer bevorzugten Ausführungsform umfasst Schritt (e) des erfindungsgemäßen Verfahrens vor dem Vereinen das Abtrennen zumindest eines Teils des Ammoniaks von dem Gasgemisch durch Abkühlen. Dazu durchläuft das Gasgemisch bevorzugt zunächst einen Wärmetauscher und anschließend eine Kondensationsvorrichtung. Dabei wird das Gasgemisch abgekühlt. Das Gasgemisch wird bevorzugt auf Temperaturen von weniger als -15°C, bevorzugter weniger als -25°C und insbesondere ca. -35°C abgekühlt, bevorzugt allerdings nicht weniger als -79°C, so dass Ammoniak unter den gegebenen Bedingungen auskondensiert und so von der Gasphase durch Phasentrennung abgetrennt werden kann. Da diese Abtrennung ggf. jedoch nicht vollständig erfolgt, enthält das im Kreislauf geführte Gasgemisch üblicherweise zumindest noch Spuren von Ammoniak.

In Schritt (f) des erfindungsgemäßen Verfahrens wird das Gasgemisch in einen zusätzlichen Ammoniakreaktor geleitet. In diesem zusätzlichen Ammoniakreaktor wird bei einem höheren Synthesedruck als in Schritt (c) Ammoniak synthetisiert aus zumindest einem Teil des Wasserstoffs und aus zumindest einem Teil des Stickstoffs, welcher in dem Gasgemisch enthalten ist. Vorzugsweise weist der zusätzliche Ammoniakreaktor zumindest ein Katalysatorbett auf, welches von dem Gasgemisch nicht rein axial, sondern vorwiegend radial durchströmt wird, vorzugsweise von außen nach innen. Bevorzugt wird dabei das Katalysatorbett nicht gekühlt, sondern die Synthese adiabat geführt. Danach wird zumindest ein Teil des synthetisierten Ammoniaks von dem Gasgemisch durch Abkühlen abgetrennt, wozu das Gasgemisch bevorzugt zunächst mindestens einen Wärmetauscher und anschließend eine Kondensationsvorrichtung durchläuft. Dabei wird das Gasgemisch abgekühlt. Das Gasgemisch wird bevorzugt auf Temperaturen von weniger als -15°C, bevorzugter weniger als -25°C und insbesondere ca. -35°C abgekühlt, bevorzugt allerdings nicht weniger als -79°C, so dass Ammoniak unter den gegebenen Bedingungen auskondensiert und so von der Gasphase durch Phasentrennung abgetrennt werden kann. Danach wird das Gasgemisch erneut auf einen erhöhten Druck verdichtet, vorzugsweise auf einen Druck im Bereich von 150 bis 280 bar, und als im Kreislauf geführtes Gasgemisch zu Schritt (e) zurückgeführt.

Bevorzugt wird in Schritt (f) des erfindungsgemäßen Verfahrens das Gasgemisch in einem geschlossenen Kreislauf zu Schritt (e) zurückgeführt, wobei aus dem geschlossenen Kreislauf Ammoniak und ggf. weitere Substanzen wie beispielsweise im Ammoniak gelöster Wasserstoff und Stickstoff oder Restmengen an Inertgas unter den Bedingungen der Ammoniakabtrennung abgetrennt werden, jedoch nicht in einem separaten Schritt Spülgas *(Purge)* ausgeschleust wird, welches Inerten enthält. Bei dieser bevorzugten Ausführungsform wird demnach über Schritt (e) weiteres Gasgemisch dem geschlossenen Kreislauf zugeführt und die Ammoniakabtrennung ist der einzige Schritt, bei dem Bestandteile aus dem Gasgemisch ausgeschleust werden, insbesondere Ammoniak, ggf. aber auch vergleichsweise geringe Mengen anderer Substanzen wie Wasserstoff und/oder Stickstoff und/oder Methan und/oder Argon und/oder Helium.

Es wurde überraschend gefunden, dass durch das erfindungsgemäße Verfahren, insbesondere durch den bevorzugten Schritt (b), die Anreicherung von Inertgas in dem im Kreislauf geführten Gasgemisch soweit verringert bzw. unterdrückt werden kann, dass auf ein separates Ausschleusen *(Purge)* verzichtet werden kann, ohne dass gleichzeitig die ansonsten mit der Anreicherung der Inertgase verbundenen Nachteile in Kauf genommen werden müssen. Bevorzugt wird die Konzentration an Kohlenmonoxid, an Kohlendioxid und an Wasser in Schritt (b) soweit verringert, dass der Katalysator, welcher zur Synthese von Ammoniak verwendet wird, nicht beeinträchtigt wird. So reicht die ggf. erfolgende Ausschleusung der Inertgase zusammen mit dem Ammoniak im Zuge der Abtrennung des Ammoniaks durch Abkühlen in Schritt (f) aus, ggf. vorhandene Restmengen an Inertgas aus dem System auszuschleusen und auf diese Weise den Gehalt an Inertgas in dem im Kreislauf geführten Gasgemisch dauerhaft verschwindend gering zu halten. Als eine Folge davon können bei höherer Ausbeute an Ammoniak geringere Katalysatorvolumina eingesetzt werden, was das Verfahren insgesamt wirtschaftlicher macht. Auch eine deutlich kleinere Bauweise der Apparate, Rohrleitungen und Armaturen wird möglich.

Die vorgenannte Variante des Verfahrens ist diejenige ohne Purge. Alternativ dazu können Inertgase (in der vorliegenden Erfindung Edelgase und Methan) in höherer Konzentration vorhanden sein und separat mit Spülgas ausgeschleust werden. Dieses Ausschleusen mit Spülgas bedeutet, dass immer auch alle anderen Gase, die sich in dem Gasgemisch befinden, mit ausgeschleust werden. Somit werden sowohl Eduktgase (H₂, N₂) als auch das Produktgas NH₃ mit aus dem Kreislauf ausgeschleust, was zur Folge hat, dass man entweder einen Anteil Eduktgase und Produktgas verliert oder aber diese durch aufwändige nachgeschaltete Aufbereitungsschritte aus dem abgeführten Gasgemisch zurückgewinnen muss. Aus diesem Grunde ist es natürlich vorteilhaft, wenn man ohne Purge auskommt.

Ferner wurde überraschend gefunden, dass das erfindungsgemäße Verfahren so betrieben werden kann, dass das Gasgemisch bereits nach dem ersten Verdichter, den das Gasgemisch durchströmt, ggf. in Schritt (c) oder auch vorher, auf einen Druck im Bereich von vorzugsweise 150 bis 180 bar verdichtet werden kann. In diesem Fall kommt das Verfahren ggf. insgesamt mit zwei Verdichterstufen für Schritte (c) und (d) sowie einer weiteren Verdichterstufe im Kreislauf für Schritt (f) zur Überwindung von Druckverlusten aus, wohingegen herkömmliche Verfahren mindestens eine weitere Verdichterstufe erforderlich machen.

Schritte (a) bis (f) des erfindungsgemäßen Verfahrens werden vorzugsweise in alphabetischer Reihenfolge durchgeführt, jedoch nicht zwingend unmittelbar im Anschluss aneinander. So ist es durchaus möglich und auch bevorzugt, dass vor, zwischen oder nach einzelnen Schritten oder innerhalb einzelner Schritte weitere Maßnahmen erfolgen. Bevorzugt wird in Schritt (c) und/oder in Schritt (d) des erfindungsgemäßen Verfahrens das Gasgemisch vor dem Verdichten zunächst ggf. stark abgekühlt und erst danach verdichtet. Die Abkühlung des Gasgemischs erfolgt dabei, sobald wasserfrei, bevorzugt mit Wärmetauschern auf Temperaturen unterhalb von 0°C, bevorzugt auf -16°C oder weniger. Es wurde überraschend gefunden, dass sich auf diese Weise die Kapazität der Anlage im Hinblick auf die Gesamtausbeute an Ammoniak erheblich steigern lässt, da bei geringerer Eingangstemperatur in den Ammoniakreaktor eine größere Stoffmenge an Wasserstoff und Stickstoff in den Ammoniakreaktor gelangt und zu Ammoniak umgesetzt werden kann. Bevorzugt wird die bei der Abkühlung des Gasgemischs freigesetzte Wärme teilweise dazu genutzt, um Wasserdampf zu erzeugen und/oder um Kesselspeisewasser vorzuwärmen.

Gemäß einer bevorzugten Weiterbildung der vorliegenden Erfindung wird das Gasgemisch vor dem Verdichten durch Einspritzen von Ammoniak getrocknet. Durch diese Maßnahme wird schon vor der ersten Sythesegas-Kompressor-Stufe Wasser abgeschieden, so dass man das Frischgas vor den ersten beiden Stufen auf Tieftemperaturen von beispielsweise in der Größenordnung von etwa -30 °C abkühlen (chillen) kann. Damit wird der Synthesegaskompressor deutlich entlastet und ein höherer Gasdurchsatz wird ermöglicht. Außerdem wird durch das Anreichern mit Ammoniak erreicht, dass die Synthese-Reaktion im ersten Bett des OT-Konverters (once-through) ein wenig gehemmt wird und dadurch die Gefahr der Überhitzung und der Schädigung des Katalysators reduziert wird.

In einer bevorzugten Ausführungsform erfolgt das Synthetisieren von Ammoniak in Ammoniaksyntheseeinheiten, welche ein oder mehrere Katalysatorbetten umfassen, wobei das Gasgemisch zwischen den Katalysatorbetten abkühlt. Bevorzugt wird die zwischen den Katalysastorbetten durch Abkühlung freigesetzte Wärme teilweise genutzt, um Wasserdampf zu erzeugen und/oder um Kesselspeisewasser vorzuwärmen.

In einer besonders bevorzugten Ausführungsform wird Schritt (c) des erfindungsgemäßen Verfahrens vor der Durchführung von Schritt (d) mindestens einmal wiederholt, ggf. auch häufiger, z.B. zweimal, dreimal oder viermal, wobei bei jeder Wiederholung von Schritt (c) das Gasgemisch auf einen Druck verdichtet wird, welcher höher ist als der Druck zuvor in Schritt (c), und wobei danach in Schritt (d) das Gasgemisch auf einen Druck verdichtet wird, welcher höher ist als der Druck bei der letzten Wiederholung von Schritt (c). Um die Wiederholung von Schritt (c) zu ermöglichen, erfolgt die Durchführung vorzugsweise mit Hilfe mehrerer, hintereinandergeschalteter Ammoniaksyntheseeinheiten, wobei die Ammoniakreaktoren der Ammoniaksyntheseeinheiten bevorzugt alle gemeinsam in einem Druckbehälter oder jeweils einzeln in mehreren, hintereinandergeschalteten Druckbehältern angeordnet sind. Bevorzugt wird das Gasgemisch jeweils zwischen dem Durchlaufen der einzelnen Ammoniakreaktoren abgekühlt, um Ammoniak teilweise abzutrennen und einen höheren Umsatz an Ammoniak zu erzielen. Geeignete Reaktoren sind einem Fachmann bekannt, beispielsweise aus EP 1 339 641 B1.

Bevorzugt weist das in Schritt (a) des erfindungsgemäßen Verfahrens bereitgestellte Gasgemisch ein relatives molares Verhältnis von Wasserstoff zu Stickstoff von mehr als 3:1 auf, vorzugsweise von mehr als 5:1, und der bevorzugte Schritt (b) des erfindungsgemäßen Verfahrens umfasst nach dem Abtrennen das Anreichern des Gasgemischs mit Stickstoff, bevorzugt bis auf ein relatives molares Verhältnis von Wasserstoff zu Stickstoff von ca. 3:1, wie es für die anschließende Ammoniaksynthese wünschenswert ist. Bevorzugt wird das Gasgemisch mit Stickstoff angereichert, welcher durch eine Luftzerlegung bereitgestellt wird.

Bevorzugt wird in Schritt (a) des erfindungsgemäßen Verfahrens das bereitgestellte Gasgemisch durch Reformierung von Kohlenwasserstoffen, bevorzugt von Erdgas hergestellt, wobei die Reformierung in einem autothermen Reformer erfolgt, welcher bevorzugt mit reinem Sauerstoff, mit an Sauerstoff angereicherter Luft oder mit Luft betrieben wird. Bevorzugt wird in Schritt (a) das hergestellte Gasgemisch einer CO-Konvertierung und anschließend einer Kohlendioxidwäsche unterzogen. Geeignete Maßnahmen sind einem Fachmann bekannt. Der reine Sauerstoff, die an Sauerstoff angereicherte Luft bzw. die Luft kann dabei direkt dem autothermen Reformer zugeführt werden, wobei es auch möglich ist, dass zunächst eine Mischung mit Wasser und/oder Kohlenwasserstoffen, vorzugsweise mit Erdgas erfolgt. Diese Maßnahmen führen insgesamt zu einer Entlastung des *FrontEnd* der Ammoniakanlage, d.h. zu einer Entlastung der Erzeugung des Synthesegases (Gasgemischs), welches in Schritt (a) des erfindungsgemäßen Verfahrens bereitgestellt wird. Autotherme Reformer sind erfindungsgemäß bevorzugt, da die alternativen zweistufigen Dampf-Methan-Reformer bei hohen Anlagenkapazitäten und hohen Drücken konstruktionsbedingt unwirtschaftlich sind.

Bevorzugt erfolgt die Reformierung bei erhöhtem Druck, so dass das erzeugte Gasgemisch (Synthesegas), welches in Schritt (a) bereitgestellt wird, bereits einen vergleichsweise hohen Druck aufweist, beispielsweise von mindestens 70 bar, bevorzugter mindestens 80 bar und insbesondere mindestens 90 bar. Der erhöhte Reformierungsdruck hat u.a. auch den Vorteil, dass bei der Reformierung eine größere Menge an Kohlendioxid anfällt, welches im Anschluss an die Herstellung von Ammoniak mit Ammoniak bevorzugt zu Harnstoff umgesetzt wird. Bevorzugt ist der Reformer ein autothermer Reformer, welcher bevorzugt bei einem Druck von mindestens 100 bar betrieben wird. Bei einer Reformierung bei erhöhtem Druck ergibt sich ggf. der Nachteil eines hohen Methan-Gehalts in dem bei der Reformierung erzeugten Gasgemisch. Dieser Nachteil kann ggf. dadurch kompensiert werden, dass das Gasgemisch nach einer Stickstoffwäsche im Schritt (b) direkt für die Synthese von Ammoniak verwendet werden kann und nicht weiter verdichtet werden muss.

Es wurde überraschend gefunden, dass bei dem erfindungsgemäßen Verfahren eine Abtrennung zumindest eines Teils des ursprünglich enthaltenen Kohlendioxids wirtschaftlich vorteilhaft durch physikalische CO₂-Wäsche durchgeführt werden kann. Eine CO₂-Wäsche ist bevorzugt Bestandteil der Gasreinigung, welche bei der Herstellung von Synthesegas aus Kohlenwasserstoffen nach der Reformierung durchlaufen wird. Da sich mit steigendem Druck und sinkenden Temperaturen die Effizienz der physikalischen CO₂-Wäsche im Vergleich zur chemischen CO₂-Wäsche verbessert, umfasst das erfindungsgemäße Verfahren bevorzugt eine zumindest teilweise Abtrennung des in dem Gasgemisch enthaltenen Kohlendioxid durch physikalische CO₂-Wäsche. Geeignete Maßnahmen zur chemischen und physikalischen CO₂-Wäsche sind einem Fachmann bekannt. Bevorzugt ist eine solche CO₂-Wäsche einer CO-Konvertierung nachgeschaltet.

In einer bevorzugten Ausführungsform werden die in Schritt (b) abgetrennten Teile
(i) den Kohlenwasserstoffen beigemischt, welche im Reformer reformiert werden; oder
(ii) in wenigstens drei Teilströme aufgeteilt, wobei ein erster Teilstrom an Kohlenmonoxid, ein zweiter Teilstrom an Kohlendioxid und ein dritter Teilstrom an Methan angereichert ist, und wobei
   - der erste Teilstrom eine CO-Konvertierung durchläuft;
   - der zweite Teilstrom dem bei einer Kohlendioxidwäsche gebildeten Kohlendioxidstrom beigemischt wird; und
   - der dritte Teilstrom den Kohlenwasserstoffen beigemischt wird, welche im Reformer reformiert werden.

Bevorzugt werden die Schritte (i) und (ii) kombiniert. Bevorzugt wird der dritte Teilstrom den Kohlenwasserstoffen beigemischt, welche im Reformer reformiert werden.

Wird in Schritt (a) des erfindungsgemäßen Verfahrens das Gasgemisch einer Kohlendioxidwäsche unterzogen, wird der zweite Teilstrom bevorzugt dem Kohlendioxidstrom dieser Kohlendioxidwäsche beigemischt.

Das Aufteilen der in Schritt (b) abgetrennten Teile kann über alle einem Fachmann bekannten Aufteilvorrichtungen erfolgen. Dabei werden die abgetrennten Teile bevorzugt so aufgeteilt, dass der erste Teilstrom einen Gehalt an Kohlenmonoxid von mindestens 90 Vol.-% aufweist, bevorzugter von mindestens 95 Vol.-% oder von mindestens 99 Vol.-%. Bevorzugt weist der zweite Teilstrom einen Gehalt an Kohlendioxid von mindestens 90 Vol.-% auf, bevorzugter von mindestens 95 Vol.-% oder von mindestens 99 Vol.-%. Bevorzugt weist der dritte Teilstrom einen Gehalt an Methan von mindestens 90 Vol.-% auf, bevorzugter von mindestens 95 Vol.-% oder von mindestens 99 Vol.-%. Bevorzugt werden die Teilströme nach ihrer Aufteilung verdichtet. Bevorzugt liegen die in Schritt (b) abgetrennten Teile flüssig vor. Falls die in Schritt (b) abgetrennten Teile flüssig vorliegen, erfolgt die Verdichtung bevorzugt mit Pumpen. Die geringe Temperatur der in Schritt (b) abgetrennten Teile kann zum Kühlen von Prozessen innerhalb des erfindungsgemäßen Verfahrens oder von Prozessen anderer Verfahren verwendet werden. Beispielsweise kann die geringe Temperatur der in Schritt (b) abgetrennten Teile zum Kühlen des Gasgemischs im Zuge der Abtrennung des Ammoniaks in Schritt (f) verwendet werden.

Bevorzugt werden durch Luftzerlegung Sauerstoff und Stickstoff bereitgestellt, wobei zumindest ein Teil des Sauerstoffs dem Reformer gasförmig zugeführt wird und zumindest ein Teil des flüssigen Stickstoffs zum Abkühlen im bevorzugten Schritt (b) eingesetzt wird.

Der Ammoniakreaktor in Schritt (c), der weitere Ammoniakreaktor in Schritt (d) und/oder der zusätzliche Ammoniakreaktor in Schritt (d) weisen bevorzugt jeweils zumindest ein Katalysatorbett auf, welche von den Gasgemischen jeweils nicht rein axial, sondern vorwiegend radial durchströmt werden, vorzugsweise von außen nach innen. Die erfindungsgemäß besonders bevorzugte Kombination aus
- der Bereitstellung des Gasgemischs in Schritt (a) durch autotherme Reformierung von Kohlenwasserstoffen, vorzugsweise aus Erdgas, Wasser in Form von Dampf und Sauerstoff oder mit Sauerstoff angereicherter Luft aus einer Luftzerlegungsanlage; und
- der Abtrennung zumindest eines Teils des Wassers, ggf. zumindest eines Teils des Methans und ggf. zumindest eines Teils des Argons von dem Gasgemisch in Schritt (b) durch Abkühlen, bevorzugt mit kryogenen Methoden, insbesondere durch Stickstoffwäsche,
ist bei Verfahrensführung auf unterschiedlichen Druckniveaus insbesondere dann von wirtschaftlichem Vorteil, wenn die Anlagenkapazitäten vergleichsweise sehr hoch sind, z.B. eine Herstellung von mindestens 4000 Tagestonnen Ammoniak ermöglichen, so dass große Gasmengen umgesetzt werden.

Um eine mit hohen Anlagenkapazitäten einhergehende Steigerung der Druckverluste zu vermeiden und dadurch u.a. die Stufe des Synthesegasverdichters zu entlasten, welche das im Kreislauf geführte Gasgemisch verdichtet, sind radial durchströmte Katalysatorbetten im Vergleich zu axial durchströmten Katalysatorbetten von Vorteil. Bevorzugt werden die Katalysatorbetten auch nicht gekühlt, sondern die Synthese wird bevorzugt adiabat geführt. Dies ermöglicht eine bessere Prozesssteuerung, insbesondere bei hohen Anlagenkapazitäten von z.B. mindestens 4000 Tagestonnen Ammoniak.

Ferner ist es erfindungsgemäß bevorzugt, das Gasgemisch vor jeder Verdichterstufe abzukühlen (*Chillen*). Wird als Kühlmittel Ammoniak eingesetzt, so erfolgt eine Abkühlung beispielsweise auf ∼ -30°C. Wird als Kühlmittel Stickstoff eingesetzt, so kann das Gasgemisch auf noch tiefere Temperaturen abgekühlt werden. Diese Vorkühlung entlastet die nachfolgende Verdichterstufe und ermöglicht, die Durchsatzmengen an Gas zu erhöhen, was insbesondere auch im Hinblick auf hohe Anlagenkapazitäten von z.B. mindestens 4000 Tagestonnen Ammoniak von Vorteil ist.

Im Hinblick auf erfindungsgemäß bevorzugte Anlagenkapazitäten von mindestens 4000 Tagestonnen Ammoniak kann das zweite Gehäuse des Verdichters für das Gasgemisch für Schritt (d) ggf. limitierend sein. Um dieses zu entlasten, ist es bevorzugt, möglichst viel Ammoniak in Schritt (c) von dem Gasgemisch abzutrennen. Dies gelingt insbesondere bei erhöhtem Druck, welcher die Ammoniak-Synthese begünstigt, da dann die Kondensation des Ammoniaks bereits bei höheren Temperaturen einsetzt. Bei höherem Druck verschlechtert sich im Gegenzug allerdings die Effizienz der Reformierung - der Gehalt an nicht reformiertem Methan im in Schritt (a) bereitgestellten Gasgemisch steigt an. Das erfindungsgemäße Verfahren hat nun den Vorteil, dass die Abtrennung des Methans in Schritt (b) so effizient erfolgen kann, beispielsweise durch Stickstoffwäsche, dass eine kapazitätsbedingte Erhöhung des Gehalts an Methan kein Problem darstellt, sondern problemlos durch die Abtrennung in Schritt (b) abgefangen werden kann.

Eine weitere bevorzugte Möglichkeit der Entlastung des Synthesegasverdichters in Schritt (d) wird erreicht, wenn auf eine Trocknungseinheit zur Abtrennung von Wasser vor einer, üblicherweise der zweiten, Verdichterstufe verzichtet wird. Eine solche Trocknungseinheit ist vergleichsweise kostenintensiv und bringt unerwünschte Druckverluste mit sich. Durch Schritt (b) des erfindungsgemäßen Verfahrens werden Inertgase und/oder Katalysatorgifte wie Wasserdampf ggf. bereits abgetrennt. Bevorzugt wird durch das Abtrennen von Inerten wie Argon und Methan aus dem Gasgemisch in Schritt (b) der Synthesegasverdichter in Schritt (d) zusätzlich entlastet.

Eine mögliche Variante des erfindungsgemäßen Verfahrens führt bei Durchführung des bevorzugten Schritts (b) im Vergleich zu herkömmlichen Verfahren zu einer deutlichen Entlastung des Synthesegasverdichters. Ferner wird die in Schritten (e) und (f) im Kreislauf geführte Gasmenge deutlich reduziert, da sich keine Inertgase anreichern. Dies führt gleichzeitig zu einer geringeren Anreicherung von Ammoniak vor dem Kreislaufreaktor und auf die Ausschleusung von Spülgas *(Purge)* kann vollständig verzichtet werden. Dies führt insgesamt zu einer deutlichen Steigerung der möglichen Kapazität der Anlage, ohne dass dazu die einzelnen Elemente größer dimensioniert werden müssen. Jedoch ist alternativ ebenso gut auch die Aussschleusung von Spülgas (Variante mit Purge) möglich.

In einer bevorzugten Ausführungsform
- wird das in Schritt (a) bereitgestellte Gasgemisch durch Reformierung von Kohlenwasserstoffen hergestellt, welche kein Argon oder nur eine geringe Menge an Argon aufweisen; und
- erfolgt die Reformierung in einem autothermen Reformer, welcher mit reinem Sauerstoff oder mit einem Sauerstoff/Stickstoff-Gemisch, welches kein Argon oder nur eine geringe Menge an Argon aufweist, betrieben wird, welche durch eine Luftzerlegung bereitgestellt werden; und
- wird das Gasgemisch in Schritt (b) einer CO-Konvertierung und einer Kohlendioxidwäsche unterzogen; und
- wird ggf. im Gasgemisch verbliebenes Kohlenmonoxid und oder Kohlendioxid durch Methanisierung zu Methan umgesetzt und das Methan in Schritt (b) durch Abkühlung vom Gasgemisch abgetrennt; und
- wird nach dem Abtrennen in Schritt (b) das Gasgemisch mit Stickstoff angereichert, wobei der Stickstoff durch eine Luftzerlegung bereitgestellt wird.

Ein weiterer Aspekt der Erfindung betrifft ein Verfahren zur Herstellung von Harnstoff, umfassend das Verfahren zur Herstellung von Ammoniak, wobei zumindest ein Teil des im erfindungsgemäßen Verfahren hergestellten Ammoniaks mit Kohlendioxid zu Harnstoff umgesetzt wird. Bevorzugt werden mindestens 40 Vol.-% des im erfindungsgemäßen Verfahren hergestellten Ammoniaks mit Kohlendioxid zu Harnstoff umgesetzt, bevorzugter mindestens 50 Vol.-%, mindestens 60 Vol.-%, mindestens 70 Vol.-%, mindestens 80 Vol.-%, mindestens 90 Vol.-% oder mindestens 99 Vol.-%. Ebenfalls bevorzugt wird das gesamte im erfindungsgemäßen Verfahren hergestellte Ammoniak mit Kohlendioxid zu Harnstoff umgesetzt.

In einer bevorzugten Ausführungsform wird das in Schritt (a) bereitgestellte Gasgemisch durch Reformierung von Kohlenwasserstoffen in einem autothermen Reformer unter Bildung von Kohlendioxid hergestellt, und das im erfindungsgemäßen Verfahren hergestellte Ammoniak wird zumindest zum Teil mit dem im autothermen Reformer gebildeten Kohlendioxid in Harnstoff umgesetzt. Bei der Reformierung von Kohlenwasserstoffen in einem autothermen Reformer kann die Menge an Kohlendioxid, welche bei der Reformierung anfällt, durch Variation von Reaktionsparametern wie beispielsweise dem Druck oder Dampf/Kohlenstoff-Verhältnis kontrolliert werden. Bevorzugt wird bei der autothermen Reformierung gerade so viel Kohlendioxid hergestellt, dass das im erfindungsgemäßen Verfahren hergestellte Ammoniak bevorzugt vollständig mit Kohlendioxid zu Harnstoff umgesetzt werden kann.

Ein weiterer Aspekt der Erfindung betrifft eine Vorrichtung zur Herstellung von Ammoniak umfassend die Merkmale von Anspruch 7. In einer Ausführungsform umfasst die Vorrichtung die folgenden, miteinander in Wirkverbindung stehenden Elemente:
(a) eine Vorrichtung zur Bereitstellung eines Gasgemischs umfassend Wasserstoff, Stickstoff, Wasser, Methan, Kohlenmonoxid, Kohlendioxid und ggf. Argon und ggf. Helium; einen autothermen Reformer zum Erzeugen des Gasgemisches aus Kohlenwasserstoffen, vorzugsweise Erdgas, Wasser und Sauerstoff;
(b) eine CO-Konvertierungseinheit zum Konvertieren von CO zu CO₂;
   eine CO₂-Wäsche zum Abtrennen von Kohlendioxid aus dem Gasgemisch ggf. eine Methanisierungseinheit zum Umwandeln von Kohlenmonoxid zu Methan;
   ggf. eine Abtrennungsvorrichtung zum Abtrennen von zumindest einen Teil des Methans, Wassers und Argons, ggf. zusätzlich auch zumindest einem Teil des CO bzw. CO₂ von dem Gasgemisch, vorzugsweise durch N₂-Wäsche;
   ggf. einen oder mehrere Wärmetauscher zum Abkühlen des Gasgemischs;
   ggf. eine Luftzerlegungsanlage, Mittel zum Überführen von Sauerstoff oder von mit Sauerstoff angereicherter Luft zum autotermen Reformer sowie Mittel zum Überführen von Stickstoff zur Abtrennungsvorrichtung;
   ggf. eine Vorrichtung zur Einstellung des molaren Verhältnisses von Wasserstoff zu Stickstoff auf einen Wert von etwa 3;
(c) Mittel zum Anreichern des Gasgemischs mit Stickstoff;
(d) eine oder mehrere, hintereinandergeschaltete Ammoniaksyntheseeinheiten, welche bevorzugt jeweils einen Verdichter zum Verdichten des Gasgemischs auf erhöhten Druck; einen Ammoniakreaktor zum Synthetisieren von Ammoniak aus zumindest einem Teil des Wasserstoffs und aus zumindest einem Teil des Stickstoffs, welcher in dem Gasgemisch enthalten ist; einen oder mehrere Wärmetauscher zum Abkühlen des Gasgemischs und eine Kondensierungsvorrichtung zum Abtrennen zumindest eines Teils des Ammoniaks umfassen; wobei im Falle mehrerer, hintereinandergeschalteter Ammoniaksyntheseeinheiten die bevorzugt vorhandenen Verdichter dazu ausgelegt sind, den Druck des Gasgemischs jeweils im Vergleich zur vorgeschalteten Ammoniaksyntheseeinheit zu erhöhen; und wobei im Falle mehrerer, hintereinandergeschalteter Ammoniaksyntheseeinheiten die Ammoniakreaktoren bevorzugt in einem gemeinsamen Druckbehälter oder jeweils einzeln in mehreren, hintereinandergeschalteten Druckbehältern angeordnet sind;
(e) einen Verdichter, welcher dazu ausgelegt ist, das Gasgemisch auf einen Druck zu verdichten, welcher höher ist als der Druck in der/den zuvor durchlaufenen Ammoniaksyntheseeinheit/en;
   ggf. einen weiteren Ammoniakreaktor zum Synthetisieren von Ammoniak aus zumindest einem Teil des Wasserstoffs und aus zumindest einem Teil des Stickstoffs, welcher in dem Gasgemisch enthalten ist;
   ggf. einen oder mehrere Wärmetauscher zum Abkühlen des Gasgemischs und eine Kondensierungsvorrichtung zum Abtrennen zumindest eines Teils des Ammoniaks umfassen;
(f) eine Vorrichtung zum Vereinen des Gasgemischs mit einem im Kreislauf geführten Gasgemisch umfassend Wasserstoff, Stickstoff und Ammoniak und ggf. Inerte;
(g) eine Ammoniaksyntheseeinheit umfassend einen zusätzlichen Ammoniakreaktor zum Synthetisieren von Ammoniak aus zumindest einem Teil des Wasserstoffs und aus zumindest einem Teil des Stickstoffs, welcher in dem Gasgemisch enthalten ist, einen oder mehrere Wärmetauscher zum Abkühlen des Gasgemischs, und eine oder mehrere Kondensierungsvorrichtungen zum Abtrennen zumindest eines Teils des Ammoniaks; einen Verdichter zum Verdichten des Gasgemischs auf einen höheren Druck, um den Druckverlust im Kreislauf zu kompensieren; und Mittel zum Zurückführen des Gasgemisch im Kreislauf zu der Vorrichtung zum Vereinen des Gasgemischs (f),
   sowie ggf. eine Einrichtung zum Ausschleusen von Spülgas.

Die ggf. vorhandenen Elemente der erfindungsgemäßen Vorrichtung sind bevorzugt, müssen jedoch unabhängig voneinander nicht zwingend vorhanden sein. Die Elemente der erfindungsgemäßen Vorrichtung stehen miteinander in Wirkverbindung, d.h. die erfindungsgemäße Vorrichtung umfasst geeignete Mittel zum Überführen des Gasgemischs von einem Element zum nächsten, beispielsweise geeignete Rohrleitungen.

In einer bevorzugten Ausführungsform weist zumindest die erste der (c) ggf. mehreren, hintereinandergeschalteten Ammoniaksyntheseeinheiten keinen Verdichter zum Verdichten des Gasgemischs auf erhöhten Druck auf, bevorzugt die ggf. danach angeordneten Ammoniaksyntheseeinheiten hingegen schon, wobei jeder Verdichter jeweils dem Ammoniakreaktor vorgeschaltet ist. In einer anderen bevorzugten Ausführungsform weisen alle Ammoniaksyntheseeinheiten jeweils einen Verdichter zum Verdichten des Gasgemischs auf erhöhten Druck auf, welcher den Ammoniakreaktoren jeweils vorgeschaltet ist.

Bei einer möglichen alternativen Variante weist der durch (f) Ammoniaksyntheseeinheit und Verdichter geführte Kreislauf keinen *Purge* auf, über den bei herkömmlichen Vorrichtungen zur Ammoniaksynthese angereicherte Inertgase ausgeschleust werden.

In einer bevorzugten Ausführungsform umfasst die Vorrichtung zumindest eine Ammoniaksyntheseeinheit, welche mindestens ein Katalysatorbett umfasst, welches vorwiegend radial durchströmt wird, vorzugsweise von außen nach innen.

Ein weiterer Aspekt der Erfindung betrifft eine Vorrichtung zur Herstellung von Harnstoff, umfassend die erfindungsgemäße Vorrichtung zur Herstellung von Ammoniak und die zusätzlich miteinander in Wirkverbindung stehenden Elemente:
(h) eine Harnstoffsyntheseeinheit, welche einen Harnstoffsynthesereaktor zum Synthetisieren von Harnstoff aus Ammoniak und Kohlendioxid umfasst, wobei der Ammoniak in der Vorrichtung zur Herstellung von Ammoniak hergestellt wird; und
(i) Mittel zum Überführen von Ammoniak aus der Vorrichtung zur Herstellung von Ammoniak zur Vorrichtung zur Herstellung von Harnstoff.

Geeignete Harnstoffsynthesereaktoren zum Synthetisieren von Harnstoff sind einem Fachmann bekannt. Bevorzugt werden mindestens 40 Vol.-% des in der Vorrichtung zur Herstellung von Ammoniak hergestellten Ammoniaks im Harnstoffsynthesereaktor zu Harnstoff umgesetzt, bevorzugter mindestens 50 Vol.-%, mindestens 60 Vol.-%, mindestens 70 Vol.-%, mindestens 80 Vol.-%, mindestens 90 Vol.-% oder mindestens 99 Vol.-%. Bevorzugt wird der gesamte in der Vorrichtung zur Herstellung von Ammoniak hergestellte Ammoniak im Harnstoffsynthesereaktor zu Harnstoff umgesetzt.

In einer bevorzugten Ausführungsform umfasst die Vorrichtung zur Bereitstellung eines Gasgemischs einen autothermen Reformer, in welchem mehr Kohlendioxid als in einem Dampfreformer gebildet wird.

Bevorzugt kann die Menge an Kohlendioxid, welche in einem autothermen Reformer gebildet wird, durch Variation von Reaktionsparametern wie beispielsweise dem Druck oder dem Dampf/Kohlenstoff-Verhältnis kontrolliert werden. Bevorzugt wird im autothermen Reformer gerade so viel Kohlendioxid gebildet, dass das in der Vorrichtung zur Herstellung von Ammoniak hergestellte Ammoniak bevorzugt vollständig mit Kohlendioxid zu Harnstoff umgesetzt werden kann.

Alle bevorzugten Ausführungsformen, welche vorstehend im Zusammenhang mit dem erfindungsgemäßen Verfahren beschrieben wurden, gelten analog auch im Hinblick auf die Auslegung und Gestaltung der erfindungsgemäßen Vorrichtung und werden daher diesbezüglich nicht wiederholt.

Die erfindungsgemäße Vorrichtung eignet sich besonders zur Durchführung des erfindungsgemäßen Verfahrens. Ein weiterer Aspekt der Erfindung betrifft daher die Verwendung der erfindungsgemäßen Vorrichtung zur Durchführung des erfindungsgemäßen Verfahrens.

Ein weiterer Aspekt der Erfindung betrifft ein Verfahren zur Herstellung von Ammoniumnitrat, umfassend das Verfahren zur Herstellung von Ammoniak, wobei zumindest ein Teil des im erfindungsgemäßen Verfahren hergestellten Ammoniaks zur Herstellung von Ammoniumnitrat verwendet wird. Bevorzugt werden mindestens 40 Vol.-% des im erfindungsgemäßen Verfahren hergestellten Ammoniaks zur Herstellung von Ammoniumnitrat verwendet, bevorzugter mindestens 50 Vol.-%, mindestens 60 Vol.-%, mindestens 70 Vol.-%, mindestens 80 Vol.-%, mindestens 90 Vol.-% oder mindestens 99 Vol.-%. Ebenfalls bevorzugt wird das gesamte im erfindungsgemäßen Verfahren hergestellte Ammoniak zur Herstellung von Ammoniumnitrat verwendet.

Ein weiterer Aspekt der Erfindung betrifft ein Verfahren zur Herstellung von Salpetersäure, umfassend das Verfahren zur Herstellung von Ammoniak, wobei zumindest ein Teil des im erfindungsgemäßen Verfahren hergestellten Ammoniaks zur Herstellung von Salpetersäure verwendet wird. Bevorzugt werden mindestens 40 Vol.-% des im erfindungsgemäßen Verfahren hergestellten Ammoniaks zur Herstellung von Salpetersäure verwendet, bevorzugter mindestens 50 Vol.-%, mindestens 60 Vol.-%, mindestens 70 Vol.-%, mindestens 80 Vol.-%, mindestens 90 Vol.-% oder mindestens 99 Vol.-%. Ebenfalls bevorzugt wird das gesamte im erfindungsgemäßen Verfahren hergestellte Ammoniak zur Herstellung von Salpetersäure verwendet.

Die Erfindung wird anhand der Figuren beispielhaft illustriert. Ein Fachmann erkennt, dass bei einer erfindungsgemäßen Vorrichtung nicht zwingend alle abgebildeten Merkmale gleichzeitig verwirklicht sein müssen.

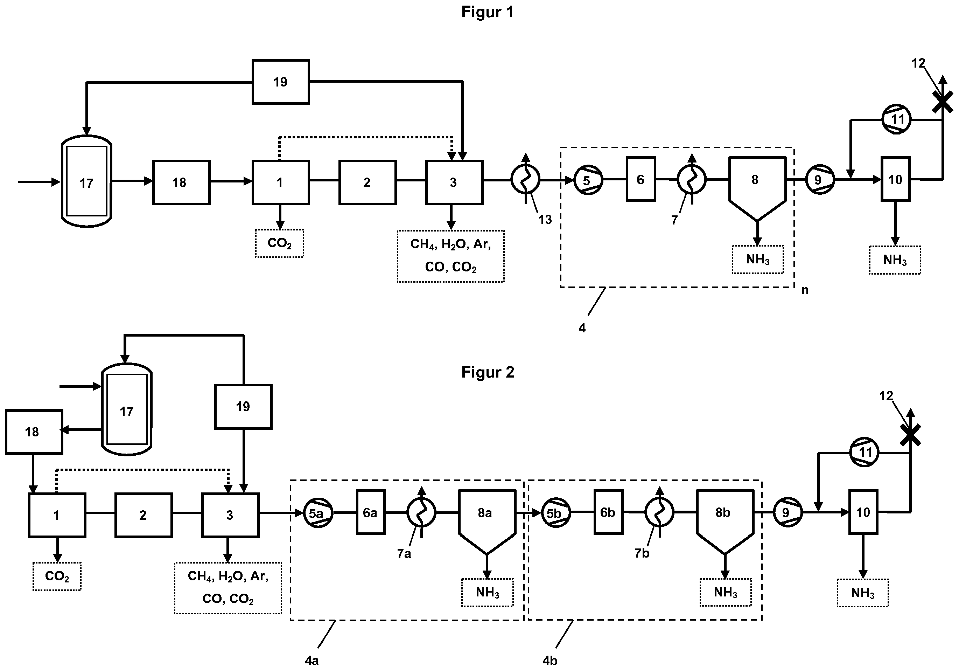

Figur 1 illustriert eine erste beispielhafte Variante einer erfindungsgemäßen Vorrichtung, mit deren Hilfe das erfindungsgemäße Verfahren durchgeführt werden kann. Dabei wird ein Gasgemisch umfassend H₂, N₂, H₂O (Dampf), CH₄, Ar, CO, CO₂ und ggf. weitere Bestandteile wie z.B. He in einem autothermen Reformer (17) bereitgestellt, danach einer CO-Konvertierung in einer CO-Konvertierungsanlage (18) unterzogen, ehe es der CO₂-Wäsche (1) zum Abtrennen von CO₂ zugeführt wird. Der autotherme Reformer (17) wird dabei mit reinem Sauerstoff oder mit an Sauerstoff angereicherter Luft betrieben, welcher in einer Luftzerlegungsanlage (19) gewonnen wird. Der reine Sauerstoff bzw. die an Sauerstoff angereicherte Luft kann dabei direkt dem autothermen Reformer zugeführt werden, wobei es auch möglich ist, dass zunächst eine Mischung mit Wasser und/oder Kohlenwasserstoffen, vorzugsweise mit Erdgas erfolgt. Das verbleibende Gasgemisch, welches ggf. noch Reste an CO₂ enthält, wird anschließend optional in eine Methanisierungseinheit (2) geleitet, in der CO und Reste von CO₂ zu CH₄ umgewandelt werden. Danach wird das bereitgestellte Gasgemisch, welches ggf. u.a. noch Reste an CO enthält, in eine Abtrennungsvorrichtung (3) überführt, in welcher zumindest ein überwiegender Teil des CH₄, H₂O und Ar, ggf. zusätzlich auch zumindest ein überwiegender Teil des CO bzw. CO₂ von dem Gasgemisch abgetrennt werden, vorzugsweise durch N₂-Wäsche, wobei der in der Luftzerlegungsanlage (19) gewonnene flüssige Stickstoff der Abtrennungsvorrichtung (3) zugeführt wird. In einer bevorzugten Variante wird die Methanisierung ausgelassen, so dass das bereitgestellte Gasgemisch von der CO₂-Wäsche (1) direkt in die Abtrennungsvorrichtung (3) überführt wird, was in den Figuren durch den gestrichelten Pfeil angedeutet ist.

Danach wird das auf tiefe Temperaturen gekühlte Gasgemisch in eine oder mehrere, hintereinandergeschaltete NH₃-Syntheseeinheiten (4) überführt, welche jeweils einen Verdichter (5), einen NH₃-Reaktor (6), einen oder mehrere Wärmetauscher (7) und eine Kondensierungsvorrichtung (8) zum Abtrennen von NH₃ umfassen. Die NH₃-Syntheseeinheit (4) ist in Figur 1 gestrichelt umrahmt. Index n kann bevorzugt 1, 2, 3, 4 oder 5 sein und bringt damit zum Ausdruck, dass im Falle von n>1 mehrere solcher NH₃Syntheseeinheiten (4) sequentiell hintereinander geschaltet sind. Im Verdichter (5) wird das Gasgemisch auf einen erhöhten Druck verdichtet und anschließend in den NH₃-Reaktor (6) überführt, in dem zumindest ein Teil des in dem Gasgemisch enthaltenen H₂ und zumindest ein Teil des in dem Gasgemisch enthaltenen N₂ zu NH₃ reagieren. Das den NH₃-Reaktor (6) verlassende Gasgemisch wird danach im Wärmetauscher (7) auf eine vergleichsweise tiefe Temperatur abgekühlt, so dass zumindest ein Teil des im Gasgemisch enthaltenen NH₃ in der Kondensierungsvorrichtung (8) auskondensiert und von dem restlichen Gasgemisch abgetrennt wird. Dabei können unter den Bedingungen der NH₃-Abtrennung ggf. auch andere Substanzen aus dem Gasgemisch mit abgetrennt werden. Sind mehrere NH₃-Syntheseeinheiten (4) hintereinandergeschaltet, so wird im Verdichter (5) der Druck des Gasgemischs jeweils im Vergleich zur zuvor durchlaufenen NH₃-Syntheseeinheit (4) erhöht, so dass die NH₃-Synthese im NH₃-Reaktor (6) der zuerst durchlaufenen NH₃-Syntheseeinheit (4) bei geringerem Druck erfolgt als die NH₃-Synthese im NH₃-Reaktor (6) der danach durchlaufenen NH₃-Syntheseeinheit/en (4).

Nach der Auskondensierung und Abtrennung zumindest eines Teils des im Gasgemisch enthaltenen NH₃ in der Kondensierungsvorrichtung (8) der zuletzt durchlaufenen NH₃-Syntheseeinheit (4) wird das Gasgemisch ggf. in Verdichter (9) auf einen Druck verdichtet, welcher höher als der Druck in der/den zuvor durchlaufenen NH₃-Syntheseeinheit/en (4) ist. Das verdichtete Gasgemisch wird dann mit einem im Kreislauf geführten Gasgemisch umfassend H₂, N₂ und NH₃ vereint und in eine NH₃-Syntheseeinheit (10) überführt, in welcher in einem zusätzlichen NH₃-Reaktor zumindest ein Teil des in dem Gasgemisch enthaltenen H₂ und zumindest ein Teil des in dem Gasgemisch enthaltenen N₂ zu NH₃ reagieren, und das Gasgemisch danach auf eine vergleichsweise tiefe Temperatur abgekühlt wird, so dass zumindest ein Teil des im Gasgemisch enthaltenen NH₃ auskondensiert und von dem restlichen Gasgemisch abgetrennt wird. Dabei können unter den Bedingungen der NH₃-Abtrennung ggf. auch andere Substanzen, z.B. Restmengen an Inertgas, aus dem Gasgemisch mit abgetrennt werden. Das verbleibende Gasgemisch wird in Verdichter (11) verdichtet, um Druckverluste im Kreislauf zu überwinden, und als im Kreislauf geführtes Gasgemisch umfassend H₂, N₂ und NH₃ mit frischem Gasgemisch vereint, ehe es erneut in eine NH₃-Syntheseeinheit (10) überführt wird.

Dabei weist der durch NH₃-Syntheseeinheit (10) und Verdichter (11) geführte Kreislauf bevorzugt keinen *Purge* (12) auf, über den bei herkömmlichen Vorrichtungen zur NH₃-Synthese angereicherte Inertgase ausgeschleust werden. Auf einen solchen *Purge* (12) kann hingegen bei der erfindungsgemäßen Vorrichtung bzw. dem erfindungsgemäßen Verfahren verzichtet werden, da die Inertgase bereits zuvor von dem Gasgemisch abgetrennt werden, insbesondere in der Abtrennungsvorrichtung (3).

Figur 2 illustriert schematisch einen Sonderfall der in Figur 1 dargestellten Vorrichtung. Index n=2, so dass zwei NH₃-Syntheseeinheiten (4) sequentiell hintereinander geschaltet sind. Die NH₃-Syntheseeinheit (4a) wird vom Gasgemisch zuerst durchlaufen und vom Verdichter (5a) auf einen erhöhten Druck verdichtet, und die NH₃-Syntheseeinheit (4b) wird vom Gasgemisch danach durchlaufen und vom Verdichter (5b) auf einen erhöhten Druck verdichtet, welcher höher als der Druck im Verdichter (5a) ist. Auf diese Weise wird der Druck des Gasgemischs sukzessive erhöht.

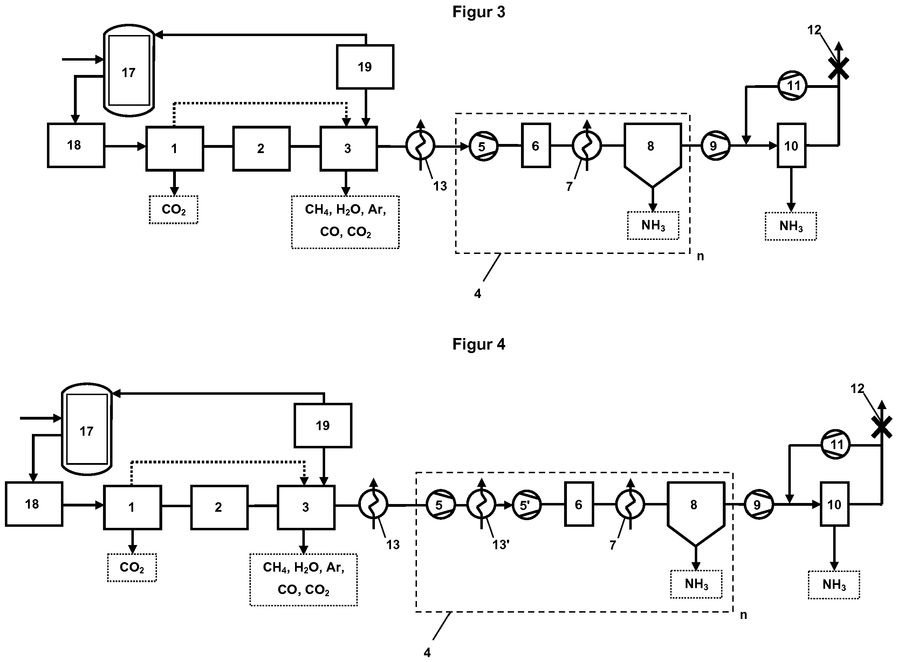

Figur 3 illustriert schematisch eine bevorzugte Variante der in Figur 1 bzw. Figur 2 dargestellten Vorrichtung, bei welcher der jeweiligen Verdichterstufe (5) der (ersten) NH₃-Syntheseeinheit (4) ein Wärmetauscher (13) vorgeschaltet ist, in dem das Gasgemisch vor der Verdichtung im (ersten) Verdichter (5) abgekühlt wird.

Figur 4 illustriert schematisch eine bevorzugte Variante der in Figur 3 dargestellten Vorrichtung, bei welcher der Abtrennungsvorrichtung (3) ein Wärmetauscher (13) nachgeschaltet ist und dann in jeder der n NH₃-Syntheseeinheit/en (4) dem jeweiligen NH₃-Reaktor (6) ein Verdichter (5), gefolgt von einem weiteren Wärmetauscher (13') sowie einem weiteren Verdichter (5') vorgeschaltet ist. Bei dieser bevorzugten Ausführungsform sind demnach zwei Verdichterstufen mit einer Zwischenkühlung einem Reaktor vorgeschaltet.

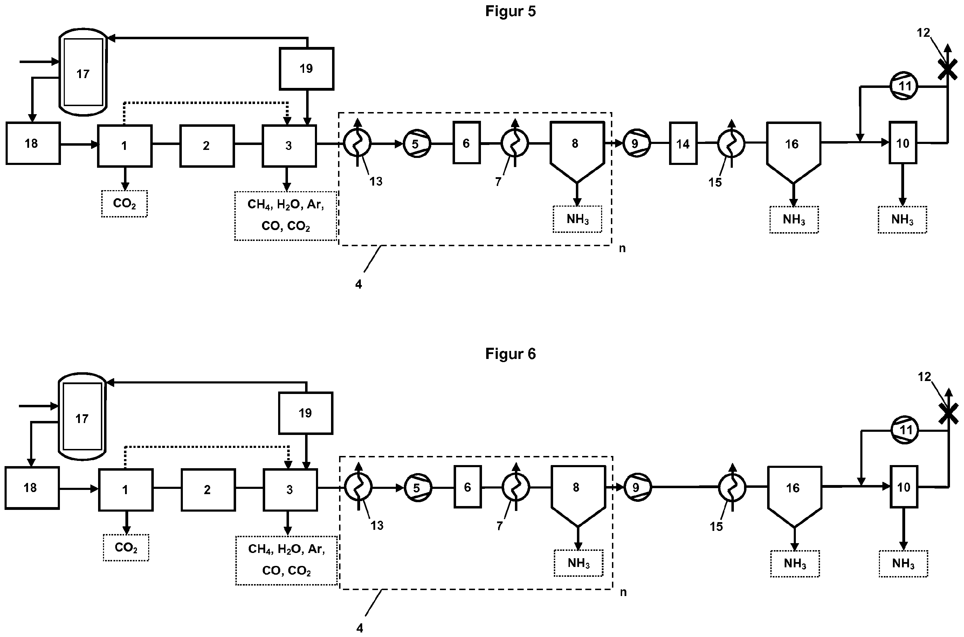

Figur 5 illustriert schematisch eine bevorzugte Variante der in Figur 4 dargestellten Vorrichtung, bei welcher zwischen Verdichter (9) und NH₃-Syntheseeinheit (10) ein weiterer NH₃-Reaktor (14), ein weiterer Wärmetauscher (15) und eine weitere Kondensierungsvorrichtung (16) angeordnet sind, die von dem Gasgemisch nacheinander durchströmt werden, ehe es mit dem im Kreislauf geführten Gasgemisch umfassend H₂, N₂ und NH₃ vereint und der NH₃-Syntheseeinheit (10) zugeführt wird.

Figur 6 illustriert schematisch eine bevorzugte Variante der in Figur 4 dargestellten Vorrichtung, bei welcher zwischen Verdichter (9) und NH₃-Syntheseeinheit (10) zwar kein weiterer NH₃-Reaktor (vgl. Figur 6), jedoch ein weiterer Wärmetauscher (15) und eine weitere Kondensierungsvorrichtung (16) angeordnet sind, die von dem Gasgemisch nacheinander durchströmt werden, ehe es mit dem im Kreislauf geführten Gasgemisch umfassend H₂, N₂ und NH₃ vereint und der NH₃-Syntheseeinheit (10) zugeführt wird.

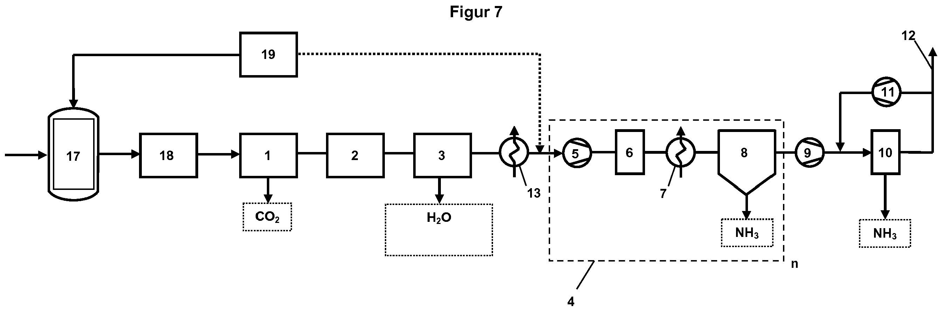

Figur 7 illustriert schematisch eine Variante der in Figur 1 dargestellten Vorrichtung, bei der in der Methanisierungseinheit (2) CO und Reste von CO₂ zu CH₄ umgewandelt werden und in der Abtrennungsvorrichtung (3) im Wesentlichen nur H₂O abgetrennt wird. Ein Teil des in der Luftzerlegungsanlage (19) gewonnenen Stickstoffs kann zusammen mit dem Sauerstoff dem autothermen Reformer (17) und ein Teil direkt zum Kompressor (5) zugeführt werden, so dass das für die NH3-Synthese Verhältnis H2:N2 etwa 3 beträgt. In dieser Variante beinhaltet das Gasgemisch nach dem Schritt (b) die Inertgase CH₄ und Ar und ggf. He. Damit diese im Synthese-Kreislauf nicht angereichert werden, erfolgt das Ausschleusen (Purge) eines Teiles des Kreislaufgases (12).

## Patentansprüche

1. Verfahren zur Herstellung von Ammoniak umfassend die Schritte
(a) Bereitstellen eines Gasgemischs umfassend Wasserstoff, Stickstoff, Wasser, Methan, ggf. Argon, Kohlenmonoxid und Kohlendioxid;
(b) Abtrennen zumindest eines Teils des Wassers, ggf. zumindest eines Teils des Methans, zumindest eines Teils des Kohlenmonoxids, zumindest eines Teils des Kohlendioxids, ggf. zumindest eines Teils des Argons von dem Gasgemisch;
(c) Synthetisieren von Ammoniak aus zumindest einem Teil des Wasserstoffs und aus zumindest einem Teil des Stickstoffs, welcher in dem Gasgemisch enthalten ist; und Abtrennen zumindest eines Teils des Ammoniaks von dem Gasgemisch durch Abkühlen;
(d) Verdichten des Gasgemischs auf einen Druck, welcher höher ist als der Druck in Schritt (c);
(e) Vereinen des Gasgemischs mit einem im Kreislauf geführten Gasgemisch umfassend Wasserstoff, Stickstoff und Ammoniak sowie gegebenenfalls die in Schritt (b) nicht vollständig abgetrennten anderen Komponenten;
(f) Synthetisieren von Ammoniak aus zumindest einem Teil des Wasserstoffs und aus zumindest einem Teil des Stickstoffs, welcher in dem Gasgemisch enthalten ist; Abtrennen zumindest eines Teils des Ammoniaks von dem Gasgemisch durch Abkühlen; Verdichten des Gasgemischs auf einen erhöhten Druck; und Zurückführen des Gasgemischs als im Kreislauf geführtes Gasgemisch zu Schritt (e)
wobei das in Schritt (a) bereitgestellte Gasgemisch durch Reformierung von Kohlenwasserstoffen hergestellt wird, wobei die Reformierung in einem autothermen Reformer erfolgt, welcher mit reinem Sauerstoff oder mit an Sauerstoff angereicherter Luft oder mit Luft betrieben wird.

2. Das Verfahren nach Anspruch 1, wobei das Gasgemisch vor dem Verdichten durch Einspritzen von Ammoniak getrocknet wird.

3. Das Verfahren nach einem der vorstehenden Ansprüche, wobei das in Schritt (a) bereitgestellte Gasgemisch in Schritt (b) u.a. einer CO-Konvertierung und einer Kohlendioxidwäsche unterzogen wird und anschließend Reste von Kohlenmonoxid und/oder Kohlendioxid durch Methanisierung weitgehend zu Methan umgesetzt werden.

4. Das Verfahren nach einem der vorstehenden Ansprüche, wobei die Reformierung in einem autothermen Reformer erfolgt, welcher mit einem Druck von mindestens 100 bar betrieben wird.

5. Das Verfahren nach einem der vorstehenden Ansprüche, wobei die in Schritt (b) abgetrennten Teile
(i) den Kohlenwasserstoffen beigemischt werden, welche im autothermen Reformer reformiert werden; oder
(ii) in wenigstens drei Teilströme aufgeteilt werden, wobei ein erster Teilstrom an Kohlenmonoxid, ein zweiter Teilstrom an Kohlendioxid und ein dritter Teilstrom an Methan angereichert ist, und wobei
- der erste Teilstrom eine CO-Konvertierung durchläuft;
- der zweite Teilstrom dem bei einer Kohlendioxidwäsche gebildeten Kohlendioxidstrom beigemischt wird; und
- der dritte Teilstrom den Kohlenwasserstoffen beigemischt wird, welche im Reformer reformiert werden.

6. Das Verfahren nach einem der vorstehenden Ansprüche, wobei das durch Schritt (b) erhaltene Gasgemisch einen
- Gehalt an Wasser von höchstens 0,05 Mol.-%; und/oder
- Gehalt an Kohlendioxid von höchstens 5ppm vol.; und/oder
- Gehalt an Kohlenmonoxid von höchstens 5 ppm vol.; und/oder
- Gehalt an Methan bis zu 3,5 Mol.-%; und/oder
- Gehalt an Argon bis zu 1,5 Mol.-%
aufweist.

7. Vorrichtung zur Herstellung von Ammoniak umfassend die miteinander in Wirkverbindung stehenden Elemente
(a) eine Vorrichtung zur Bereitstellung eines Gasgemischs umfassend Wasserstoff, Stickstoff, Wasser, Methan, Kohlenmonoxid, Kohlendioxid und ggf. Argon;
(b) eine Abtrennungsvorrichtung (3) zum Abtrennen zumindest eines Teils des Wassers, ggf. zumindest eines Teils des Methans, zumindest eines Teils des Kohlenmonoxids, zumindest eines Teils der Kohlendioxids, und ggf. zumindest eines Teils des Argons von dem Gasgemisch;
(c) Mittel zum Anreichern des Gasgemischs mit Stickstoff;
(d) eine oder mehrere, hintereinandergeschaltete Ammoniaksyntheseeinheiten (4), welche jeweils einen Ammoniakreaktor (6) zum Synthetisieren von Ammoniak aus zumindest einem Teil des Wasserstoffs und aus zumindest einem Teil des Stickstoffs, welcher in dem Gasgemisch enthalten ist; einen oder mehrere Wärmetauscher (7) zum Abkühlen des Gasgemischs und eine Kondensierungsvorrichtung (8) zum Abtrennen zumindest eines Teils des Ammoniaks umfassen;
(e) einen Verdichter (9), welcher dazu ausgelegt ist, das Gasgemisch auf einen Druck zu verdichten, welcher höher ist als der Druck in der/den zuvor durchlaufenen Ammoniaksyntheseeinheit/en (4);
(f) eine Vorrichtung zum Vereinen des Gasgemischs mit einem im Kreislauf geführten Gasgemisch umfassend Wasserstoff, Stickstoff und Ammoniak;
(g) eine Ammoniaksyntheseeinheit (10) umfassend einen Ammoniakreaktor zum Synthetisieren von Ammoniak aus zumindest einem Teil des Wasserstoffs und aus zumindest einem Teil des Stickstoffs, welcher in dem Gasgemisch enthalten ist, einen oder mehrere Wärmetauscher zum Abkühlen des Gasgemischs, und eine oder mehrere Kondensierungsvorrichtungen zum Abtrennen zumindest eines Teils des Ammoniaks; einen Verdichter (11) zum Verdichten des Gasgemisches auf einen höheren Druck, um den Druckverlust im Kreislauf zu kompensieren; und Mittel zum Zurückführen des Gasgemisch im Kreislauf zu der Vorrichtung zum Vereinen des Gasgemischs,
wobei die Vorrichtung zur Bereitstellung eines Gasgemischs einen autothermen Reformer umfasst.

8. Die Vorrichtung nach Anspruch 7, wobei die Abtrennungsvorrichtung in Schritt (b) u.a. eine CO-Konvertierungsanlage (18) umfasst, in der zumindest ein Teil von Kohlenmonoxid zu Wasserstoff konvertiert wird und/oder eine CO₂-Wäsche (1) umfasst, in der zumindest ein Teil von Kohlendioxid ausgewaschen wird; und/oder eine Methanisierungseinheit (2) umfasst, in der CO und Reste von CO₂ zu CH₄ umgewandelt werden.

9. Die Vorrichtung nach Anspruch 7 oder 8, wobei zumindest eine Ammoniaksyntheseeinheit mindestens ein Katalysatorbett umfasst, welches vorwiegend radial durchströmt wird.

10. Die Vorrichtung nach einem der Ansprüche 7 bis 9, wobei die Ammoniaksyntheseeinheiten (4) jeweils einen dem Ammoniakreaktor (6) vorgeschalteten Verdichter (5) zum Verdichten des Gasgemischs auf erhöhten Druck aufweisen; wobei im Falle mehrerer, hintereinandergeschalteter Ammoniaksyntheseeinheiten (4) die Verdichter (5) dazu ausgelegt sind, den Druck des Gasgemischs jeweils im Vergleich zur vorgeschalteten Ammoniaksyntheseeinheit (4) zu erhöhen.

11. Vorrichtung zur Herstellung von Harnstoff, umfassend die Vorrichtung zur Herstellung von Ammoniak nach einem der Ansprüche 7 bis 10, wobei die Vorrichtung die folgenden, miteinander in Wirkverbindung stehenden Elemente umfasst:
(h) eine Harnstoffsyntheseeinheit, welche einen Harnstoffsynthesereaktor zum Synthetisieren von Harnstoff aus Ammoniak und Kohlendioxid umfasst, wobei der Ammoniak in der Vorrichtung zur Herstellung von Ammoniak bereitgestellt wird; und
(i) Mittel zum Überführen von Ammoniak aus der Vorrichtung zur Herstellung von Ammoniak zur Vorrichtung zur Herstellung von Harnstoff.

12. Verfahren zur Herstellung von Ammoniumnitrat, umfassend das Verfahren zur Herstellung von Ammoniak nach einem der Ansprüche 1 bis 6, wobei zumindest ein Teil des in dem Verfahren hergestellten Ammoniaks zur Herstellung von Ammoniumnitrat verwendet wird.

13. Verfahren zur Herstellung von Salpetersäure, umfassend das Verfahren zur Herstellung von Ammoniak nach einem der Ansprüche 1 bis 6, wobei zumindest ein Teil des in dem Verfahren hergestellten Ammoniaks zur Herstellung von Salpetersäure verwendet wird.

## Claims

1. Process for the production of ammonia comprising the steps
(a) preparation of a gas mixture comprising hydrogen, nitrogen, water, methane, in some cases argon, carbon monoxide and carbon dioxide;
(b) removal of at least a part of the water, optionally at least a part of the methane, at least a part of the carbon monoxide, at least a part of the carbon dioxide, and optionally at least a part of the argon from the gas mixture;
(c) synthesis of ammonia from at least a part of the hydrogen and from at least a part of the nitrogen which is contained in the gas mixture; and removal of at least a part of the ammonia from the gas mixture by cooling;
(d) compression of the gas mixture to a pressure which is higher than the pressure in step (c);
(e) combination of the gas mixture with a recirculated gas mixture comprising hydrogen, nitrogen and ammonia and optionally the other components not completely removed in step (b);
(f) synthesis of ammonia from at least a part of the hydrogen and from at least a part of the nitrogen which is contained in the gas mixture; removal of at least a part of the ammonia from the gas mixture by cooling; compression of the gas mixture to an elevated pressure; and return of the gas mixture as recirculated gas mixture to step (e),
wherein the gas mixture prepared in step (a) is produced by reforming of hydrocarbons, wherein the reforming takes place in an autothermal reformer which is operated with pure oxygen or with oxygen-enriched air or with air.

2. Process according to Claim 1, wherein the gas mixture before the compression is dried by injecting ammonia.

3. Process according to one of the previous claims, wherein the gas mixture prepared in step (a) in step (b) is subjected inter alia to a CO conversion and a carbon dioxide scrubbing and then residues of carbon monoxide and/or carbon dioxide are largely converted to methane by methanation.

4. Process according to one of the previous claims, wherein the reforming takes place in an autothermal reformer which is operated with a pressure of at least 100 bar.

5. Process according to one of the previous claims, wherein the parts separated in step (b)
(i) are mixed with the hydrocarbons which are reformed in the autothermal reformer; or
(ii) split into at least three substreams, wherein a first substream is enriched in carbon monoxide, a second substream in carbon dioxide and a third substream in methane, and wherein
- the first substream passes through a CO conversion;
- the second substream is mixed with the carbon dioxide stream formed in a carbon dioxide scrubber; and
- the third substream is mixed with the hydrocarbons which are reformed in the reformer.

6. Process according to one of the previous claims, wherein the gas mixture obtained through step (b) has a
- content of water of at most 0.05 mol.%; and/or
- content of carbon dioxide of at most 5 ppm vol.; and/or
- content of carbon monoxide of at most 5 ppm vol.; and/or
- content of methane of up to 3.5 mol.%; and/or
- content of argon of up to 1.5 mol.%.

7. Device for the production of ammonia comprising the operatively connected elements
(a) a device for preparation of a gas mixture comprising hydrogen, nitrogen, water, methane, carbon monoxide, carbon dioxide and in some cases argon;
(b) a separation device (3) for the removal of at least a part of the water, optionally at least a part of the methane, at least a part of the carbon monoxide, at least a part of the carbon dioxide, and optionally at least a part of the argon from the gas mixture;
(c) means for enrichment of the gas mixture with nitrogen;
(d) one or more sequentially connected ammonia synthesis units (4), which each comprise an ammonia reactor (6) for the synthesis of ammonia from at least a part of the hydrogen and from least a part of the nitrogen which is contained in the gas mixture; one or more heat exchangers (7) for cooling the gas mixture and a condensing device (8) for removal of at least a part of the ammonia;
(e) a compressor (9), which is designed for compressing the gas mixture to a pressure which is higher than the pressure in the ammonia synthesis unit(s) (4) previously passed through;
(f) a device for combination of the gas mixture with a recirculated gas mixture comprising hydrogen, nitrogen and ammonia;
(g) an ammonia synthesis unit (10) comprising an ammonia reactor for synthesis of ammonia from at least a part of the hydrogen and from at least a part of the nitrogen which is contained in the gas mixture, one or more heat exchangers for cooling the gas mixture, and one or more condensing devices for the removal of at least a part of the ammonia; a compressor (11) for the compression of the gas mixture to a higher pressure in order to compensate for the pressure loss in the recirculation; and means for recirculating the gas mixture to the device for the combination of the gas mixture,
wherein the device for preparation of a gas mixture comprises an autothermal reformer.

8. Device according to Claim 7, wherein the separation device in step (b) comprises inter alia a CO conversion plant (18) in which at least a part of carbon monoxide is converted to hydrogen, and/or comprises a CO₂ scrubber (1) in which at least a part of carbon monoxide is scrubbed; and/or comprises a methanation unit (2) in which CO and residues of CO₂ are converted into CH₄.

9. Device according to Claim 7 or 8, wherein at least one ammonia synthesis unit comprises at least one catalyst bed with predominantly radial flow.

10. Device according to one of Claims 7 to 9, wherein the ammonia synthesis units (4) each have a compressor (5) connected upstream of the ammonia reactor (6) for the compression of the gas mixture to elevated pressure; wherein in the case of several consecutively connected ammonia synthesis units (4) the compressors (5) are designed to increase the pressure of the gas mixture in comparison to the respective upstream ammonia synthesis unit (4).

11. Device for the production of urea, comprising the device for the production of ammonia according to one of Claims 7 to 10, wherein the device comprises the following, operatively connected elements:
(h) a urea synthesis unit, which comprises a urea synthesis reactor for the synthesis of urea from ammonia and carbon dioxide, wherein the ammonia is prepared in the device for the production of ammonia; and
(i) means for transferring ammonia from the device for production of ammonia to the device for production of urea.

12. Process for the production of ammonium nitrate, comprising the process for the production of ammonia according to one of Claims 1 to 6, wherein at least a part of the ammonia produced in the process is used for the production of ammonium nitrate.

13. Process for the production of nitric acid, comprising the process for the production of ammonia according to one of Claims 1 to 6, wherein at least a part of the ammonia produced in the process is used for the production of nitric acid.

## Revendications

1. Procédé de fabrication d'ammoniac, comprenant les étapes suivantes :
(a) la préparation d'un mélange gazeux comprenant de l'hydrogène, de l'azote, de l'eau, du méthane, éventuellement de l'argon, du monoxyde de carbone et du dioxyde de carbone ;
(b) la séparation d'au moins une partie de l'eau, éventuellement d'au moins une partie du méthane, d'au moins une partie du monoxyde de carbone, d'au moins une partie du dioxyde de carbone, éventuellement d'au moins une partie de l'argon du mélange gazeux ;
(c) la synthèse d'ammoniac à partir d'au moins une partie de l'hydrogène et d'au moins une partie de l'azote qui est contenu dans le mélange gazeux ; et la séparation d'au moins une partie de l'ammoniac du mélange gazeux par refroidissement ;
(d) la compression du mélange gazeux à une pression qui est supérieure à la pression à l'étape (c) ;
(e) la réunion du mélange gazeux avec un mélange gazeux mis en circulation, comprenant de l'hydrogène, de l'azote et de l'ammoniac, ainsi qu'éventuellement les autres composants non entièrement séparés à l'étape (b) ;
(f) la synthèse d'ammoniac à partir d'au moins une partie de l'hydrogène et d'au moins une partie de l'azote qui est contenu dans le mélange gazeux ; la séparation d'au moins une partie de l'ammoniac du mélange gazeux par refroidissement ; la compression du mélange gazeux à une pression élevée ; et le recyclage du mélange gazeux en tant que mélange gazeux mis en circulation dans l'étape (e),
le mélange gazeux préparé à l'étape (a) étant fabriqué par reformage d'hydrocarbures, le reformage ayant lieu dans un reformeur autotherme qui est exploité avec de l'oxygène pur ou avec de l'air enrichi en oxygène ou avec de l'air.

2. Procédé selon la revendication 1, dans lequel le mélange gazeux est séché avant la compression par injection d'ammoniac.

3. Procédé selon l'une quelconque des revendications précédentes, dans lequel le mélange gazeux préparé à l'étape (a) est soumis à l'étape (b) entre autres à une conversion du CO et une élimination par lavage du dioxyde de carbone, puis des résidus de monoxyde de carbone et/ou de dioxyde de carbone sont essentiellement transformés en méthane par méthanisation.

4. Procédé selon l'une quelconque des revendications précédentes, dans lequel le reformage a lieu dans un reformeur autotherme, qui est exploité avec une pression d'au moins 100 bar.

5. Procédé selon l'une quelconque des revendications précédentes, dans lequel les parties séparées à l'étape (b)
(i) sont incorporées dans les hydrocarbures qui sont reformés dans le reformeur autotherme ; ou
(ii) sont divisées en au moins trois courants partiels, un premier courant partiel étant enrichi en monoxyde de carbone, un deuxième courant partiel en dioxyde de carbone et un troisième courant partiel en méthane, et
- le premier courant partiel traversant une conversion de CO ;
- le deuxième courant partiel étant incorporé dans le courant de dioxyde de carbone formé lors d'une élimination par lavage du dioxyde de carbone ; et
- le troisième courant partiel étant incorporé dans les hydrocarbures qui sont reformés dans le reformeur.

6. Procédé selon l'une quelconque des revendications précédentes, dans lequel le mélange gazeux obtenu à l'étape (b) présente :
- une teneur en eau d'au plus 0,05 % en moles ; et/ou
- une teneur en dioxyde de carbone d'au plus 5 ppm en volume ; et/ou
- une teneur en monoxyde de carbone d'au plus 5 ppm en volume ; et/ou
- une teneur en méthane de jusqu'à 3,5 % en moles ; et/ou
- une teneur en argon de jusqu'à 1,5 % en moles.

7. Dispositif pour la fabrication d'ammoniac, comprenant les éléments suivants en liaison active les uns avec les autres :
(a) un dispositif pour la préparation d'un mélange gazeux comprenant de l'hydrogène, de l'azote, de l'eau, du méthane, du monoxyde de carbone, du dioxyde de carbone et éventuellement de l'argon ;
(b) un dispositif de séparation (3) pour la séparation d'au moins une partie de l'eau, éventuellement d'au moins une partie du méthane, d'au moins une partie du monoxyde de carbone, d'au moins une partie du dioxyde de carbone et éventuellement d'au moins une partie de l'argon du mélange gazeux ;
(c) des moyens pour l'enrichissement du mélange gazeux en azote ;
(d) une ou plusieurs unités de synthèse d'ammoniac raccordées en série (4), qui comprennent chacune un réacteur de synthèse d'ammoniac (6) pour la synthèse d'ammoniac à partir d'au moins une partie de l'hydrogène et d'au moins une partie de l'azote qui est contenu dans le mélange gazeux ; un ou plusieurs échangeurs de chaleur (7) pour le refroidissement du mélange gazeux et un dispositif de condensation (8) pour la séparation d'au moins une partie de l'ammoniac ;
(e) un compresseur (9), qui est conçu pour comprimer le mélange gazeux à une pression qui est supérieure à la pression dans la ou les unités de synthèse d'ammoniac (4) traversées auparavant ;
(f) un dispositif pour la réunion du mélange gazeux avec un mélange gazeux mis en circulation, comprenant de l'hydrogène, de l'azote et de l'ammoniac,
(g) une unité de synthèse d'ammoniac (10), comprenant un réacteur de synthèse d'ammoniac pour la synthèse d'ammoniac à partir d'au moins une partie de l'hydrogène et d'au moins une partie de l'azote qui est contenu dans le mélange gazeux, un ou plusieurs échangeurs de chaleur pour le refroidissement du mélange gazeux, et un ou plusieurs dispositifs de condensation pour la séparation d'au moins une partie de l'ammoniac ; un compresseur (11) pour la compression du mélange gazeux à une pression plus élevée afin de compenser la perte de charge dans le circuit ; et des moyens pour le recyclage du mélange gazeux dans le circuit vers le dispositif pour la réunion du mélange gazeux,
le dispositif pour la préparation d'un mélange gazeux comprenant un reformeur autotherme.

8. Dispositif selon la revendication 7, dans lequel le dispositif de séparation à l'étape (b) comprend entre autres une unité de conversion de CO (18), dans laquelle au moins une partie du monoxyde de carbone est convertie en hydrogène, et/ou une élimination par lavage du CO₂ (1), dans laquelle au moins une partie du dioxyde de carbone est éliminée par lavage ; et/ou une unité de méthanisation (2), dans lequel du CO et des résidus de CO₂ sont transformés en CH₄.

9. Dispositif selon la revendication 7 ou 8, dans lequel au moins une unité de synthèse d'ammoniac comprend au moins un lit catalytique, qui est traversé essentiellement radialement.

10. Dispositif selon l'une quelconque des revendications 7 à 9, dans lequel les unités de synthèse d'ammoniac (4) comprennent chacune un compresseur (5) raccordé en amont du réacteur de synthèse d'ammoniac (6), pour la compression du mélange gazeux à une pression plus élevée ; dans le cas de plusieurs unités de synthèse d'ammoniac raccordées en série (4), les compresseurs (5) étant conçus de manière à augmenter à chaque fois la pression du mélange gazeux en comparaison de l'unité de synthèse d'ammoniac raccordée en amont (4).

11. Dispositif pour la fabrication d'urée, comprenant le dispositif pour la fabrication d'ammoniac selon l'une quelconque des revendications 7 à 10, le dispositif comprenant les éléments suivants, en liaison active les uns avec les autres :
(h) une unité de synthèse d'urée, qui comprend un réacteur de synthèse d'urée pour la synthèse d'urée à partir d'ammoniac et de dioxyde de carbone, l'ammoniac étant préparé dans le dispositif pour la fabrication d'ammoniac ; et
(i) des moyens pour le transfert d'ammoniac depuis le dispositif pour la fabrication d'ammoniac dans le dispositif pour la fabrication d'urée.

12. Procédé de fabrication de nitrate d'ammonium, comprenant le procédé de fabrication d'ammoniac selon l'une quelconque des revendications 1 à 6, au moins une partie de l'ammoniac fabriqué dans le procédé étant utilisé pour la fabrication de nitrate d'ammonium.

13. Procédé de fabrication d'acide nitrique, comprenant le procédé de fabrication d'ammoniac selon l'une quelconque des revendications 1 à 6, au moins une partie de l'ammoniac fabriqué dans le procédé étant utilisé pour la fabrication d'acide nitrique.
